# EUROPEAN PATENT SPECIFICATION

(11) **EP 1 596 766 B1**
(45) Date of publication and mention of the grant of the patent: **19.10.2016**
(21) Application number: 04704475.5
(22) Date of filing: 22.01.2004
(51) Int. Cl.: A61F 2/36, A61F 2/30

(54) **INTRINSIC STABILITY IN A TOTAL HIP STEM**
INTRINSISCHE STABILITÄT IN EINEM HÜFTGELENKSTOTALSYSTEM
STABILITE INTRINSEQUE DANS UNE TIGE DE HANCHE TOTALE

(30) Priority: 22.01.2003 US 442188 P; 31.03.2003 US 405065
(43) Date of publication of application: 23.11.2005
(73) Proprietor: Encore Medical Asset Corporation, Vista CA 92081 (US)
(72) Inventor: MCTIGHE, Timothy, Chagrin Falls, OH 44023 (US); MURRAY, Ian, P., Hunt Valley, MD 21031 (US)
(74) Representative: EIP
(86) International application number: PCT/US2004/001884
(87) International publication number: WO 2004/064676

(56) References cited:
- EP-A- 0 797 964
- EP-A2- 0 782 842
- EP-A2- 0 808 618
- EP-B1- 0 711 534
- WO-A-01/43650
- WO-A-97/33538
- FR-A1- 2 735 971
- US-A- 4 549 319
- US-A- 5 061 287
- US-A- 5 362 311
- US-A- 5 549 702
- US-A1- 2002 138 151

## Description

### BACKGROUND OF THE INVENTION

### 1. The Field of the Invention.

The present invention relates generally to prosthetic implants, and more particularly, but not necessarily entirely, to a prosthetic hip system for increasing the intrinsic stability between the prosthetic implant and at least one bone. The closest prior art is WO 9733538A, which defines the preamble of claim 1.

### 2. Description of Related Art.

It is known in the art to replace the natural hip joint with an artificial hip replacement. Numerous artificial implants are available that can be used to replace the natural hip joint with an artificial ball and socket combination. Although there are many techniques used in a hip replacement surgery to replace the natural femoral components of the hip joint, each technique essentially requires resection of the femoral head, exposing the medullary canal of the femur, and creating an enlarged medullary cavity and an enlarged medullary canal in the distal portion of the proximal femur using a reamer, such that a prosthetic femoral implant may be implanted therein.

Generally, after the proximal femur has been surgically prepared, a distal stem portion of the prosthetic femoral implant may be inserted into the reamed section of the medullary canal, and a proximal stem portion of the prosthetic femoral implant may be inserted into the enlarged cavity of the proximal femur in a secure, seated position. It will be appreciated that typical prosthetic femoral implants include at least the following: a neck member that extends medially and proximally away from the proximal stem portion of the implant and terminates in a substantially spherical head member, and a stem component. The head member is configured for being inserted into an artificial acetabular implant that is configured for being located within the acetabulum of the hip. The head member may be further configured for rotational contact with the acetabular component about the three major orthogonal axes.

There are two major systems to secure the femoral component of the implant within the medullary canal of the femur. The first system, sometimes referred to as a cementless system, utilizes the natural tendencies of the bone to grow into porous sections of the femoral implant without the aid of cement. The cement less system requires the removal of a majority, if not all, of the softer, cancellous bone and uses the natural tendencies of the bone to grow into the implant, forming a tight, secure fit between the implant and the bone, to thereby maintain the implant within said bone. This system was first introduced nearly forty years ago and has become the preferred method of installation in recent years due, at least in part, to the strength of the connection between the implant and the bone ingrowth.

The second system, sometimes referred to as a cemented system, utilizes bone cement to maintain the implant within the bone. The use of cement requires the removal of bone tissue while leaving a layer of cancellous bone tissue to anchor the implant to the bone with the aid of cement. This process was used extensively during the 1970's and 1980's, and is still commonly used today on a more limited basis in comparison with the cementless system.

Both systems may be advantageously used in appropriate circumstances depending upon a patient's needs. For example, recovery from an operation using the cementless system takes an average of about three months before the patient may return to any activity so that new bone may be permitted to grow into the pores of the implant. The result is a connection that has the potential to endure in the patient for a long period of time, for some patients that may be as long as 20 years or more. The cementless system is recommended for patients who lead active lives, and is typically used in relatively young patients.

Conversely, the cemented system results in a decrease in post-operative pain, compared to the cementlesa system, and an increase in joint mobility. However, the interface between the bone, the cement and the implant may not be as strong as the cementless system and may result in premature loosening as compared to the cementless system. Therefore, the cemented system is typically used in less active, older patients.

It is a fairly common occurrence for femoral implants to loosen from the bone or cement over time due, at least in part, to the high stresses placed on the hip joint. Specifically in cementless total hip arthroplasty, dislocation of the hip joint has been and continues to be a problem. In recent years a trend has developed in the orthopedic industry to increase the femoral offset of the implant between the head of the implant and a long axis of the femur to help reduce dislocation. As the femoral offset increases, the potential for increased torsional forces placed on the stem-bone interface likewise increases, and the potential for the stem loosening increases, resulting in increased post-operative pain, disability and an increased risk that additional revision surgery may be necessary. Attempts have been made in the prior art to increase the efficiency of the bond between the implant and either bone or cement, such that the loosening of the implant from the bone (or from the cement in cemented systems) over time is decreased.

One such attempt to improve the adhesion of the stem of the implant to the bone, or cement is found in U.S. Patent No. 5,480,452 (granted January 2, 1996 to Hofmann et al.). Hofmann et al. discloses a femoral prosthesis having a proximal portion formed as a wedge for thrusting into the medullary canal and achieving fixation to the bone, ribs for securing the prosthesis against medial-lateral motion, while providing a degree of flexibility in the anterior-posterior direction, and a slot formed in the distal stem, which is flared for enhancing fixation distally. However, this device is disadvantageous in that the device is unable to withstand The increased torsional loads that maybe placed on the device due to an increase in the lateral offset and to the fictional forces acting tangentially on the bone-implant interface. Torsional forces are disadvantageous in that over time they may cause loosening of the implant from the bone.

U.S. Patent No. 5,935, 172 (Ochoa et al.) discloses a joint prosthesis having a plurality of negative surface features and comprises a first, body portion and a second, cap portion for the distal end of the body to fit into. The body further has a metaphyseal fitting region to contact the surrounding bone to initiate bone ingrowth. However, this device is disadvantageous because it lacks the structure necessary to contact. the posterior calcar wall and the anterior cortex of the femur permitting solid contact with cortical bone. Thus, torsional forces may not be desisted.

EP-A2-0 782 842 discloses a prosthesis that includes at least two intersecting cone-like bodies whose outer surfaces engage the end of a patient's bone EP-A2-0 782 842 further discloses cavities for receiving the cone-like bodies that can be readily formed in the patient's bone with a high level of precision. Through the use of the two cone-like bodies, the prosthesis may have neutral version and yet be used at various angles without sacrificing the integrity of the patient's bone or the degree of securement of the prosthesis.

US 2002/138151 A1 discloses a cementless femoral hip stem component. The hip stem component includes an elongate stem, a proximale body, and a collar disposed on the proximal body and extending outward therefrom in a sideways direction to form a ledge. The proximal body defines at least a majority of a frustoconical shape. The ledge formed by the collar includes a tapered undersurface, beneath which extends the proximal body. The tapered undersurface of the ledge and the proximale body extend in different directions and thereby cooperatively define a double-flared contact surface with the femur at two different rates of subsidence within the femur: An abrupt, male corner is formed by the stem and the proximal body and is sufficiently abrupt to provide enhanced bone ingrowth stimulation.

The prior art is thus characterized by several disadvantages that are addressed by the present invention. The present invention minimizes, and in some aspects eliminates, the above-mentioned failures, and other problems by utilizing the methods and structural features described herein.

According to the present invention there is provided a prosthetic device for implantation into a bone as defined in claim 1.

The features and advantages of the invention will be set forth in the description which follows, and in part will be apparent from the description, or may be learned by the practice of the invention without due experimentation. The features and advantages of the invention may be realized and obtained by means of the instruments and combinations particularly pointed out in the appended claims.

### BRIEF DESCRIPTION OF THE DRAWINGS

The features and advantages of the invention will become apparent from a consideration of the subsequent detailed description presented in connection with the accompanying drawings in which:
FiG. 1 is a posterior side view of a femoral prosthetic device.
FIG. 1A is a side view of a modular neck;
FIG. 1B is a side view of an alternative modular neck;
FIG. 1C is a bottom view of the modular neck of FIG. 1B, illustrating a shape of a first and second taper;
FIG. 1D is a front view of a top portion of a proximal conical flare with the modular neck removed, for illustrating a recess formed in the top of the proximal conical flare;
FIG. 2 is a front view of the femoral prosthetic device of FIG. 1;
FIG. 3 is a posterior side view of an alternative example the femoral prosthetic device of FIG. 1;
FIG. 4 is a front view of the femoral prosthetic device of FIG. 3;
FIG. 5 is a back view of an embodiment of the femoral prosthetic device illustrating a proximal conical flare and an anterior metaphyseal tapering flare;
FIG. 6 is an anterior, partially broken side view of the femoral prosthetic device of FIG. 5 illustrating the modular neck component of the present invention;
FIG. 7 is a back view of another embodiment of the femoral prosthetic device illustrating the proximal conical flare and a restrictor made in accordance with the principles of the present invention;
FIG. 8 is an anterior, partially broken side view of the femoral prosthetic device of FIG. 7;
FIG. 9A is a side view illustrating an embodiment of the femoral prosthetic device in a varus position;
FIG. 9B is a side view similar to FIG. 9A illustrating the femoral prosthetic device in a neutral position, and also illustrating the restrictor acting as a centralizer;
FIG. 9C is a side view similar to FIGS. 9A-9B illustrating the femoral prosthetic device in a valgus position;
FIG. 10 is a back view of another embodiment of the femoral prosthetic device made in accordance with the principles of the present invention;
FIG. 11 is an anterior side view of the femoral prosthetic device of FIG. 10 illustrating the modular neck component and made in accordance with the principles of the present invention;
FIG. 12 is a back view of another embodiment of the femoral prosthetic device illustrating the anterior metaphyseal tapering flare made in accordance with the principles of the present invention;
FIG. 13 is an anterior, partially broken side view of the femoral prosthetic device of FIG. 12 illustrating the modular neck component and made in accordance with the principles of the present invention;
FIG. 14 is a back view of another example of the femoral prosthetic device illustrating the proximal conical flare;
FIG. 15 is an anterior, partially broken side view of the femoral prosthetic device of FIG. 14, and illustrating one example of a bushing insert and modular neck component;
FIG. 15A is an enlarged side view of the bushing insert of FIG. 15;
FIG. 16 is a back view of another example of the femoral prosthetic device illustrating the proximal conical flare;
FIG. 17 is an anterior, partially broken side view of the femoral prosthetic device of FIG. 16 illustrating another example of the bushing insert and modular neck component;
FIG. 18 is a back view of another example of the femoral prosthetic device illustrating the proximal conical flare;
FIG. 19 is an anterior, partially broken side view of the
   femoral prosthetic device of FIG. 18 illustrating another example of the bushing insert and modular neck component;
FIG. 19A is an enlarged view of the bushing insert and recess similar to FIG. 19, illustrating the bushing insert and recess as cylindrically shaped.
FIG. 20 is a back view of another example of the femoral prosthetic device illustrating the proximal conical flare;
FIG. 21 is an anterior side view of the femoral prosthetic device of FIG. 20;
FIG. 22 is a back view of another embodiment of the femoral prosthetic device illustrating the proximal conical flare and a helical slot;
FIG. 23 is an anterior side view of the femoral prosthetic device of FIG. 22 illustrating the modular neck component; and
FIG. 24 is a side view of a failed titanium femoral prosthetic device.

### DETAILED DESCRIPTION OF THE INVENTION

For the purposes of promoting an understanding of the principles in accordance with the invention, reference will now be made to the examples and embodiments illustrated in the drawings and specific language will be used to describe the same. It will nevertheless be understood that no limitation of the scope of the invention is thereby intended. Any alterations and further modifications of the inventive features illustrated herein, and any additional applications of the principles of the invention as illustrated herein, which would normally occur to one skilled in the relevant art and having possession of this disclosure, are to be considered within the scope of the invention as defined by the claims

Before the present device and methods are disclosed and described, it is to be understood that this invention is not limited to the particular configurations, process steps, and materials disclosed herein as such configurations, process steps, and materials may vary somewhat. It is also to be understood that the terminology employed herein is used for the purpose of describing particular embodiments only and is not intended to be limiting since the scope of the present invention will be limited only by the appended claims and equivalents thereof.

The publications and other reference materials referred to herein describe the background of the invention and provide additional detail regarding its practice. The references discussed herein are provided solely for their disclosure prior to the filing date of the present application. Nothing herein is to be construed as a suggestion or admission that the inventors are not entitled to antedate such disclosure by virtue of prior invention.

Designers of hip stem prostheses may choose to increase the lateral offset between a femoral head of an implant and the longitudinal axis, or mid-line, of a femur in order to restore, at least partially, the biomechanics of the natural hip joint. An increased lateral offset operates to increase the torsional forces that are exerted on the femoral implant, and such forces may be applied to the bone-implant interface specifically between a stem portion of the implant and the medullary canal of the femur. Additionally, torsional forces may be derived from the sum of the interface surface friction forces acting parallel to the interface surface, and the torque created by the forces normal to the interface surface acting to resist the offset force applied to the femoral head. There is, therefore, an increased need for torsional stability to prevent the implant from loosening from the bone.

Applicants have discovered that torsional forces may more effectively be opposed by utilizing a prosthetic device having a variety of intrinsic stabilization features, some of which may contact the cortical bone surfaces of the femur to aid in resisting torsional forces. Applicants have further discovered that by interchanging and combining several of the intrinsic stabilization features, different results may be achieved, thus allowing a surgeon to adjust the device to the needs of a particular patient by combining several of the intrinsic stabilization features.

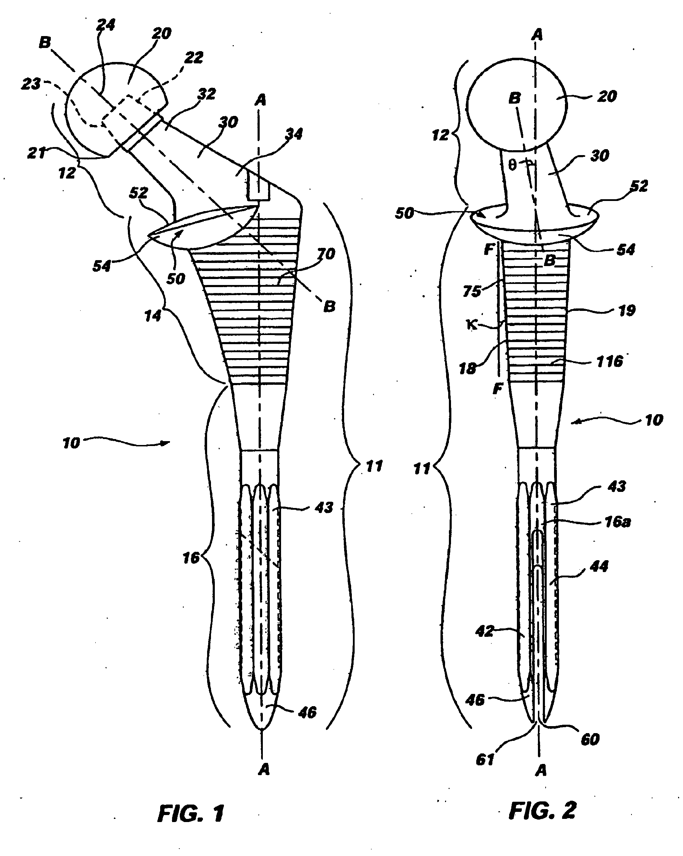

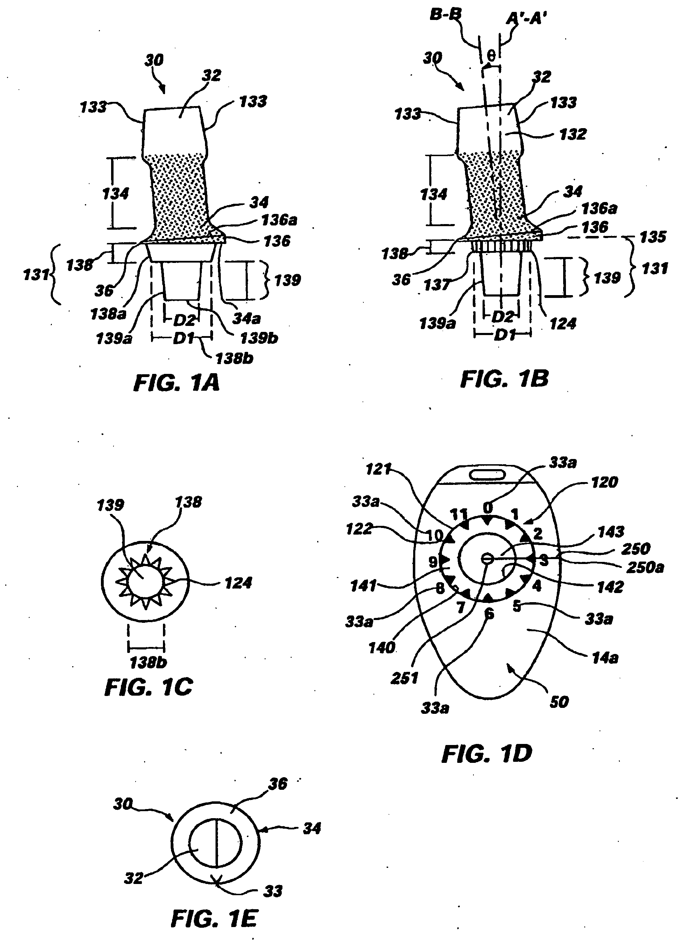

Referring now to FIG. 1, there is illustrated a femoral prosthetic device, generally designated at 10, which may be fashioned of any suitable bio-compatible material including metal, such as titanium, stainless steel, cobalt-chromium-molybdenum alloy, titanium-aluminum vanadium alloy, or other alloys thereof. FIG. 1 illustrates many of the characteristics that may be present in several embodiments of the present invention and it should be noted that like reference numerals will be used to indicate like structure in the drawings.

It will be appreciated that the femoral prosthetic device 10 of the present invention may generally be separated into two distinct portions, parts or components. Namely, a stem component 11, and a head/neck component 12. The stem component 11 may further be separated into a proximal portion 14, also referred to herein as a proximal body portion or a proximal stem portion, and a distal portion 16, also referred to herein a distal stem portion. It will be appreciated that the proximal portion 14 may comprise approximately twenty-five to fifty percent of the entire stem component 11, while the corresponding distal portion may comprise approximately fifty to seventy-five percent of the entire stem component 11, as illustrated in the FIGS. The head/neck component 12 of the femoral prosthetic device 10 may generally comprise a femoral head component 20, and a neck component 30.

It will be appreciated that the device 10 may have a longitudinal axis, designated by the line A-A, that may be centered with respect to the distal portion 16 of the stem component 11. The axis A-A may also extend centrally between a proximal end 11a and a distal end 11b of the stem component 11. A plane may run through the longitudinal axis A-A and may separate the stem component 11 into an anterior side 18 and a posterior side 19. Accordingly, the axis A-A may delineates the stem component 11 into distinct anterior 18 and posterior sides 19. It will be appreciated that the anterior side 18 and the posterior side 19 of the device 10 may be distinguished by the features of the present invention. Therefore, the device 10 may be manufactured such that each device 10 may be particularly made for being implanted into a left or right femur, to be used as part of a hip replacement.

The femoral head component 20 may act as the ball portion of the ball and socket joint and may be configured and dimensioned to attach to an acetabular bearing surface of an acetabular device, such as an acetabular cup (not illustrated in the figures), which may be used as the socket of the ball and socket joint. The femoral head component 20 may be substantially spherical, as shown, or may be any other suitable shape that is either presently known, or which may become known in the future, in the art for attaching the femoral component to the acetabular bearing surface, and that functions as the ball portion of a ball and socket joint.

It will be appreciated that the femoral head component 20 may be attached to the neck component 30 in a manner known in the art. For example, a distal end 21 of the head component 20 may include an aperture 22, illustrated as dashed lines in FIG. 1, defined by tapered sidewalls 23 for matingly engaging a matching tapered sidewall 133 of the neck component 30 defining a proximally tapered neck portion (illustrated best in FIGS. 1A and 1B) such that a locking fit may be accomplished. It should be noted that other structural features currently known, or which may become known in the future, in the art may be incorporated into the device 10 to attach the head component 20 to the neck component 30, and any of the various other features known in the art for attaching the head component 20 to the neck component 30 may be used by the present invention without departing from the scope of the present invention.

It should be noted that the neck component 30 may be configured as a modular neck 30 or as an integral neck 30 without departing from the scope of the present invention. The modularity of the neck component 30 advantageously creates an ability for the surgeon to fine tune and adjust the femoral prosthetic device 10 by increasing or decreasing the lateral offset relative to the patient's needs. Additionally, the modularity of the neck component 30 may aid the surgeon during a revision surgery without removing the entire stem component 11.

As used herein, the phrase "lateral offset" refers to the horizontal distance relative to a patient in a standing position from the center of the pelvis to the center of the femoral canal in the natural hip joint. In the prosthetic implant 10, "lateral offset" refers to the horizontal distance between a central preference 24 of the femoral head component 20 and the longitudinal axis A-A of the femoral stem component 11 of the implant 10. It will be appreciated that the lateral offset may be increased or decreased by replacing the modular neck 30 with another differently sized modular neck 30, which may be longer or shorter than the modular neck 30 being replaced. Thus, the length of the neck 30 may function to increase or decrease the lateral offset.

Referring now to FIGS. 1A and 1B, the neck component 30 may be comprised of a proximal end 32 and a distal end 34. The proximal end 32 comprises the tapered sidewall 133 for engaging the corresponding tapered sidewall of the aperture formed in the head component 20, as described above. The distal end 34 may comprise an undersurface 34a. The neck component 30 may further comprise a shaft portion 134 separating the proximal end 32 from the distal end 34. It will be appreciated that the shaft portion 134 may be lengthened or shortened to increase or decrease the overall length of the neck component 30. A tapered portion 131 may extend distally below the undersurface 34a of the distal end 34 of the modular neck component 30 and may comprise an outer tapered portion 138 extending immediately below said distal end 34 from the undersurface 34a. The tapered portion 131 may further comprise an inner tapered portion 139 extending distally below, and may essentially be disposed on, the outer tapered portion 138. The outer tapered portion 138 may have a diameter D1 that may be greater than or equal to a diameter D2 of the inner tapered portion 139. The outer tapered portion 138 may comprise an outer tapered sidewall 138a, and a plurality of first splines 124 defined within and surrounding the outer tapered sidewall 138a of the outer tapered portion 138, while the inner tapered portion 139 may also comprise an inner tapered wall 139a. It will be appreciated that the above tapered portion 131 may be referred to herein as an indexable portion comprising a dual combination of tapered wall surfaces, which may be referred to herein as a double taper.

It will be appreciated that the double taper may advantageously provide a primary lock, and a secondary lock, should the primary lock fail. Additionally, the features associated with the indexable portion 131 may also provide the surgeon with the added flexibility of assembling and disassembling the device 10 during surgery without removing the stem component 11 from the bone.

As illustrated particularly in FIG. 1B, the longitudinal axis A'-A' of the neck component 30, also referred to herein as the reference axis A' -A', when utilized in conjunction with the neck component 30, may be defined as being normal to a plane 135 of a base 36 at the distal end 34 of the neck component 30. An angle θ, also referred to herein as an anteversion angle θ, is also illustrated in FIG. 1B, and may be defined as the angle between the reference axis A'-A' and an anteverted axis B-B, also referred to herein as the neck axis B-B. Thus, the angle θ of the neck component 30 may allow the head portion 20 to be located either farther anteriorly, or farther posteriorly within the hip joint depending upon the orientation of the neck component 30 within a recess 120 of the proximal portion 14 of the stem component 11. Exemplary anteversion angles θ, found to be beneficial for a majority of patients, may be between the range of about zero and about twenty degrees, and more specifically about ten degrees. It should be noted that one of skill in the art could modify the anteversion angle θ without departing from the scope of the present invention such that the anteversion angle θ could be greater than twenty degrees, depending upon the need of the patient and the desired result.

As illustrated in FIGS. 1A and 1B, the neck component 30 may comprise an anteverted portion 136 for creating an anteversion in the neck component 30, which may be located near the base 36, on the distal end 34 of said modular neck component 30. A surface 136a of the anteverted portion 136 may taper at an angle with respect to a plane 135, and may be positioned orthogonally to the neck axis B-B creating the anteversion of the neck component 30. It should be noted that one of skill in the art may modify the angle of the anteverted portion 136 to increase or decrease the anteversion angle θ, or may reposition the anteverted portion 136 to be located on any part of the modular neck component 30 to create the desired anteversion in the neck component 30, without departing from the scope of the present invention. It should further be noted that one of skill in the art could modify the current invention, without departing from the scope of the present invention, so as to eliminate the anteverted portion 136 completely, and simply angle the shaft 134 of the neck component 30 to the desired anteversion angle θ.

It will be appreciated that the angle of anteversion θ may be adjusted. For example, as illustrated in FIG**.** 1E, a' marker 33 may be utilized to position the modular neck component 30 in varying angles of anteversion. Referring to FIGS. 1B, 1D, and 1E, when the marker 33 is positioned in alignment with a reference numeral 33a the modular neck component 30 may have a predetermined angle of anterversion θ. It will be appreciated that opposing reference numerals 33a may correspond to similar version angles θ, only the version of the modular neck component 30 will be positioned in the opposite direction, either anteriorly or posteriorly. Furthermore, when marker 33 is in alignment with reference numeral 33a labeled as number "0" or "6" (illustrated best in FIG. 1D), the modular neck component will have a zero degree anteversion angle θ.

Referring now to FIGS. 1B and 2, wherein the neck component 30 is illustrated as being anteverted as described above. It will be appreciated that the discussion above regarding anteversion and associated angles may apply to neck components 30 that may be integral or modular. For example, the anteversion angle θ of the modular neck component 30 of FIG. 1B, and the anteversion angle θ of the integral neck component 30 in FIG. 2 are both illustrated as being about ten degrees. It should be noted that the neck components 30 may have a zero degree angle of anteversion, or in other words, the angle of anteversion may not be present, as described above. The anteversion angle utilized by the present invention may be configured to simulate the natural femoral neck anteversion angle. It should be noted that the angle of anteversion may be modified by one of skill in the art to include those anteversion angles that may simulate the natural femur.

The examples of FIGS. 1A and 1B are illustrated as being generally the same with only minor distinctions. One distinction between the FIGS. occurs in the indexable portion 131 regarding the double taper. It will be appreciated that the embodiment of FIG. 1A illustrates the outer tapered portion 138 as being smooth having no grooves, splines, protuberances or gear teeth located on the taper. Whereas, the embodiment of FIG. 1B, illustrates the outer tapered portion 138 as having the plurality of first splines 124 defined within a perimeter 138b of the outer tapered sidewall 138a forming gear teeth 137 for matingly engaging a plurality of corresponding second splines 122 defined within a first sidewall defining the first portion 141 of the recess 120 of the stem component 11 (illustrated best in FIG. 1D) forming corresponding gear teeth in the recess 120. The perimeter 138b may be defined as the area bounded by the outer tapered sidewall 138a without any of the first splines 124 located thereon, similar to the outer tapered portion 138 in FIG. 1A. It should be noted that the gear teeth 137 may be tapered, as they are a part of the outer tapered portion 138. It will be appreciated that the first splines 124 of the outer tapered portion 138 may act in concert with the corresponding second splines 122 of the first portion 141 of the recess 120 of the stem component 11, permitting the modular neck 30 to be indexed in a plurality of predetermined positions and orientations. Additionally, the connection between the first splines 124 and corresponding second splines 122 may permit the surgeon to fine tune and adjust the modular neck 30 such that stress points may be altered or shifted.

It should be noted that the outer tapered portion 138 may be modified by one of skill in the art to be of any length, either larger or smaller than illustrated in FIGS. 1A and 1B. The outer tapered portion 138 may be any length presently known, or which may become known in the future, in the art for securing and orienting the neck component 30 to the stem component 11, and may further be modified to increase or decrease the angle of taper.

As illustrated in FIGS. 1A and 1H, the inner tapered portion 139 extends below the outer tapered portion 138 and may be between the range of about one to about ten times the length of the outer tapered portion 138. For example the inner tapered portion 139 may be about three to about four times the length of the outer tapered portion 138. It will be appreciated that the inner tapered portion 139 may also be equal in length to the outer tapered portion 138.

Each of the inner tapered portion 139 and the outer tapered portion 138 may utilize a taper angle relative to the reference axis A'-A', wherein the taper angle that may be within a range of self-locking tapers, and the self-locking taper of the inner tapered portion 139 and the outer tapered portion 138 may be utilized together or individually. It should be noted that the length of the inner tapered portion 139 may be such that the taper does not bottom out such that a secure connection between the neck component 30 and the stem component 11 may occur. It will be appreciated that the term "bottom out," as used herein, refers to the condition where the tapered portion 131 of the modular neck component 30, particularly a distal end 139b of the inner tapered portion 139, descends to the lowest point possible in the recess 120 of the stem component 11, which recess 120 may be formed within the proximal portion 14 of the stem component 11, before being fully seated within the recess 120, such that the primary locking fit and the self-locking taper fit does not fully occur. Therefore, it will be appreciated that the best possible connection will not occur when the tapered portion 131 bottoms out in the recess 120.

FIG. 1B illustrates the inner tapered portion 139 being longer than the example of the inner tapered portion 139 illustrated in FIG. 1A. In order for the inner tapered portion 139 of FIG. 1B to not bottom out, the corresponding recess 120 must be lengthened such that the inner tapered portion 139, and its distal end 139b, does not contact the lowest possible point of the recess 120. If the inner tapered portion 139 does contact the lowest point possible in the recess 120, the inner tapered portion 139 will bottom out and the tapered lock may not occur, or if it does occur, the tapered lock may be weakened or compromised.

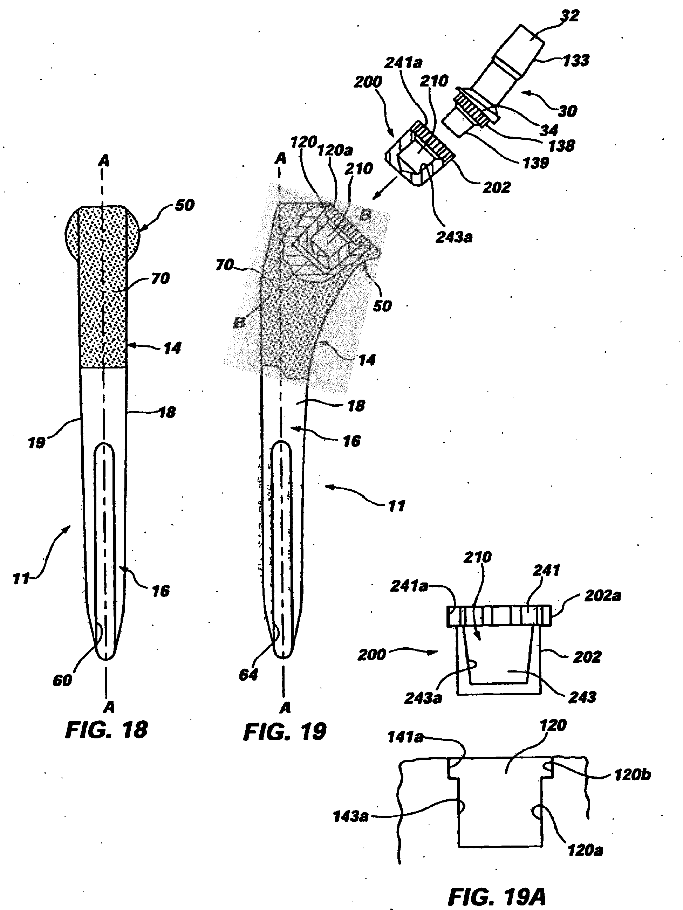

The inner tapered portion 139 may function to provide a connection with the recess 120 that acts as a primary self-locking taper for locking and securing the neck component 30 to the stem component 11. Whereas, the outer tapered portion 138 may function as a secondary locking taper to secure the neck component 30 to the stem component 11, and may act as an emergency backup to maintain the stem component 11 as part of the femoral prosthetic device 10 such that the stem component 11 does not separate from the rest of the femoral prosthetic device 10, should the primary locking taper fail for any number of reasons. It should be noted that the primary and secondary locks may be modified such that the outer tapered portion 138 provides the primary locking function, while the inner tapered portion 139 provides the secondary locking function. It will be appreciated that the outer tapered potion 138 and the inner tapered portion 139 may each be modified by one of skill in the art to be of any length, either larger or smaller than illustrated in FIGS. 1A and 18. The outer tapered portion 138 and the inner tapered portion 139 may be modified to increase or decrease the angle of taper.

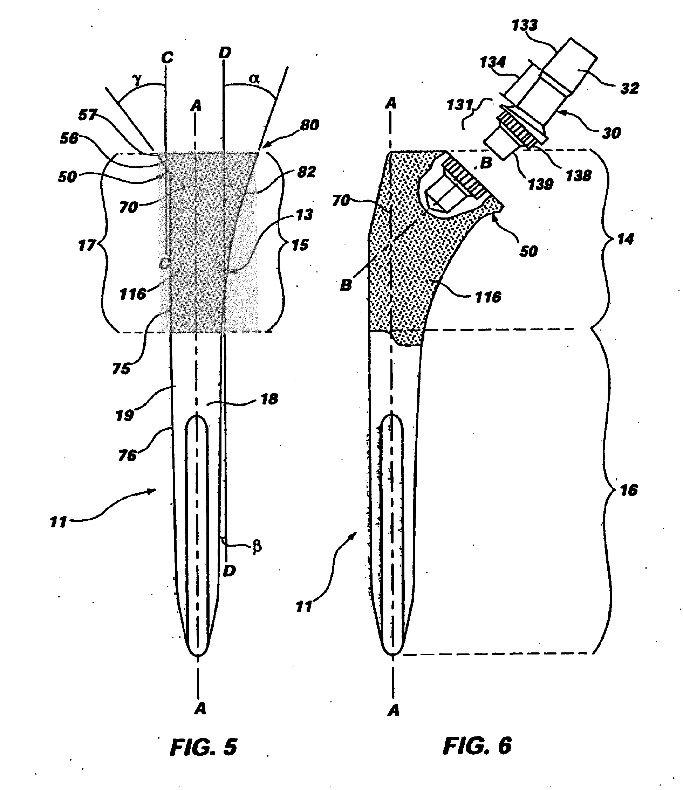

As illustrated in FIGS. 1, 1D, and 6, the proximal portion 14 of the stem component 11 may have a surface 14a configured with the recess 120 for receiving the indexable portion 131 and the double taper of the modular neck component 30. The recess 120 may be comprised of the first portion 141, which may be defined by the first sidewall 140, and a second portion 143, which may be defined by a second sidewall 142.

It will be appreciated that the recess 120 may be present when the femoral prosthetic device 10 utilizes the modular neck 30, but may not be present when the device 10 utilizes the integral neck 30. FIG. 1D illustrates a top view of the surface 14a within which the recess 120 may reside below. As mentioned previously, the first sidewall 140 may define the first portion 141 of the recess 120, and is illustrated in FIG. 1D as having corresponding second splines 122 defined within the first sidewall 140. It will be appreciated that the first splines i24 and corresponding second splines 122 may be as illustrated, or may be modified by one of skill in the art to produce second splines 122 having either a more blunt edge or a sharper edge than illustrated in FIG. 1D. It will further be appreciated that the first splines 124 and corresponding second splines 122 may be modified to include other mechanisms that function similarly to first splines 124 and corresponding second splines 122 to index the modular neck component 30 within the recess 120.

It will likewise be appreciated that the number of first splines 124 of the outer tapered portion 138 and the number of corresponding second splines 122 may also be modified to include more or less first splines 124 and corresponding second splines 122 than illustrated. It will be appreciated that as the number of splines increases or decreases in either the outer tapered portion 138 or the first portion 141 of the recess 120, the opposite and corresponding component's splines will be modified in number accordingly. It will further be appreciated that the outer tapered portion 138 may be modified to remove the first splines 124 such that the outer tapered portion 138 may be substantially smooth, and the first splines 124 may be located on the inner tapered portion 139, for example. Accordingly, the first sidewall 140 of the recess 120 may also be modified by one of skill in the art by removing the corresponding second splines 122 such that the first sidewall 140 may be a smooth sidewall to matingly engage the smooth outer tapered portion 138. The corresponding second splines 122 may be located, for example, on the second sidewall 142 of the recess 120.

As stated previously, the corresponding second splines 122 may function as gear teeth having twelve different positions or orientations, denoted by numerals 0-11 situated in a similar position as a standard clock. The differing positions may be established by the first splines 124 of the outer tapered portion 138 and the corresponding second splines 122 of the first sidewall 140. The first splines 124 and the corresponding second splines 122 may matingly engage one another in any one of the twelve positions or orientations, which permits the modular neck 30 to be arranged in a specific orientation such that differing version angles may be achieved. The version angle may be adjusted by removing the modular neck 30 from the recess 120 and rotating the modular neck 30 to the desired orientation creating the desired version angle. It should be noted that the splines and corresponding second splines 122 may be modified by one of skill in the art such that more or less than twelve different positions or orientations, by which the modular neck 30 may be attached to the recess 120, may be achieved.

FIG. 1C is a bottom view of the modular neck 30 illustrating the outer tapered portion 138 and the inner tapered portion 139. It will be appreciated that the tapered fit between the first splines 124 of the outer tapered portion 138 and the corresponding second splines 122 of the first sidewall 140 may be referred to herein as a tapered interlock.

As mentioned previously, the second sidewall 142 formed within the recess 120 may define a cavity or depression, and may further define the second portion 143. It should be noted that both the first portion 141 and the second portion 143 may be tapered at an angle relative to the neck axis B-B, wherein the taper angle may match the corresponding taper of outer tapered portion 138 and the inner tapered portion 139, respectively, of the modular neck 30, such that the recess 120 and the modular neck 30 may be locked together. Accordingly, the taper angle of the first portion 141 and the second portion 143 may be within a range of taper angles of the self-locking type, and the second portion 143 may provide for the primary fixation of the recess 120 to the modular neck 30, thus connecting the proximal portion 14 to the head/neck component 12 of the device 10.

It will be appreciated that the depth of the second portion 143 of the recess 120 may be dimensioned to be deep enough so as to avoid "bottoming out" of the taper, ensuring that the self-locking taper may fully occur. Whereas, the outer tapered portion 138 of the modular neck 30 may be configured for matingly engaging the first portion 141 of the recess 120 forming a secondary lock or fixation, should the primary lock or fixation fail.

It will be appreciated that the structure and apparatus disclosed herein is merely one example of a positioning means for positioning the modular neck component in multiple selectable orientations within the recess of the stem component, and it should be appreciated that any structure, apparatus or system for positioning the modular neck component in multiple selectable orientations, which performs functions the same as, or equivalent to, those disclosed herein are intended to fall within the scope of a positioning means for positioning the modular neck component in multiple selectable orientations, including those structures, apparatus or systems for positioning the modular neck component in multiple selectable orientations, which are presently known, or which may become available in the future.

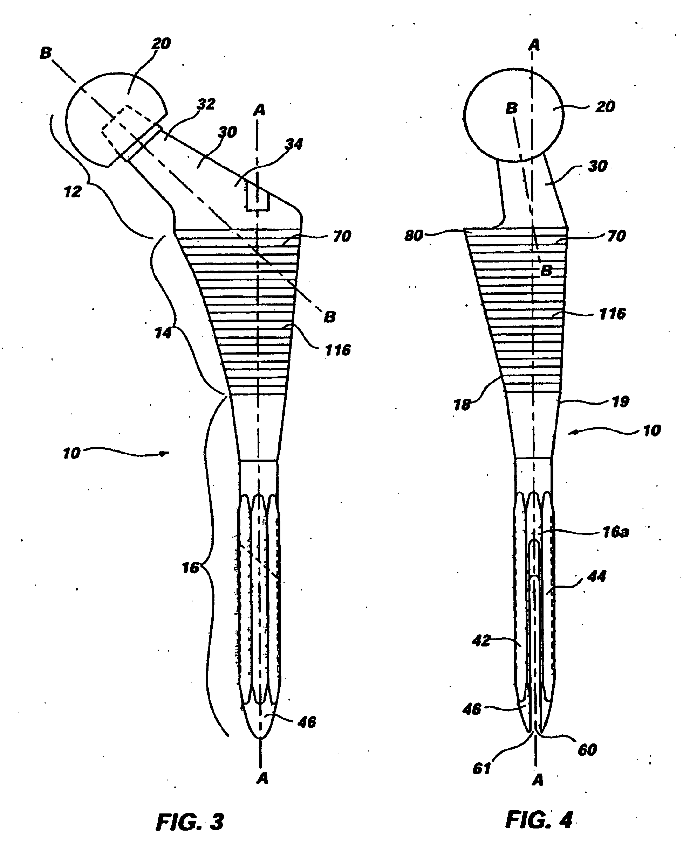

Referring back to FIG. 1, it will be appreciated that the proximal portion 14 of the stem component 11 may include various features of the present invention, some of which may include: (i) a proximal conical flare 50, including a posterior flare (ii) an anterior metaphyseal tapering flare 80, sometimes referred to herein as an anterior flare, an anatomical body or an anatomical proximal body (illustrated best in FIGS. 4 and 5), and (iii) a tapered exterior surface 75 configured to provide surface contact with a proximal. portion of the cortical bone in the femur (illustrated best in FIG. 2).

The proximal portion 14 of the present invention may comprise the proximal conical flare 50 and an enlarged proximal body portion 70 configured for filling, at least partly, the metaphyseal cavity in the femur. As illustrated, the proximal conical flare 50 may be located proximally on the proximal portion 14 of the stem component 11. Specifically, the proximal conical flare 50 may be formed near the proximal end 11a of the stem component 11, as illustrated in FIG. 5.

As illustrated in FIGS. 1 and 5, the proximal conical flare 50 may comprise an undersurface 54 having a contour that may be shaped in a rounded conical manner. The proximal conical flare 50 may extend outwardly in the anterior, posterior and medial directions. It will be appreciated that the proximal conical flare 50 may have an anterior/posterior radius 250 (illustrated best in FIG. 1D) defined as the distance between a point 251 that is central with respect to the recess 120 and an end 250a located on the anterior or posterior edge of the proximal conical flare 50. It will be appreciated that the radius on the anterior side 18 may be larger than the radius on the posterior side 19, when the anterior metaphyseal tapering flare 80 is present. The radius 250 may increase as the size of the metaphyseal cavity increases, and/or as the size of the stem component 11 increases to more completely fill the metaphyseal cavity in the bone, such that the proximal conical flare 50 increases, although such is not required.

The proximal conical flare 50 may further have a surface 56 that tapers at an angle relative to a line C-C (the line C-C being parallel to the longitudinal axis A-A) forming a posterior flare 57 that may be located proximally on the posterior side 19 of the stem component 11 such that the proximal conical flare 50 may fill at least a portion of a cavity in the bone. It will be appreciated that the posterior flare 57 may be formed from about one to about twenty percent of the entire stem component 11 on the upper most portion of the proximal portion 14. For example, applicants have found that the posterior flare 57 that comprises about four to ten percent of the entire stem component 11 to be useful, and particularly about four to six percent. The surface 56 of the posterior flare 57 may have a flare angle relative to the line C-C that is parallel to the longitudinal axis A-A, represented by γ, that may be between the range of about fifteen degrees to about forty-five degrees. For example, applicants have found that the surface 56 having a flare angle γ between the range of about twenty degrees to about forty degrees to be advantageous, and more specifically, applicants have found that a flare angle γ of thirty degrees to be advantageous.

In addition to the above range of angles for surface 56, the flare angle γ may, for example, be about fifteen degrees, or about sixteen degrees, or about eighteen degrees, or about twenty degrees, or about twenty-two degrees, or about twenty-four degrees, or about twenty-six degrees, or about twenty-eight degrees, or about thirty degrees, or about thirty-two degrees, or about thirty-four degrees, or about thirty-six degrees, or about thirty-eight degrees, or about forty degrees, or about forty-two degrees, or about forty-four degrees, or about forty-five degrees.

The posterior flare 57 may be configured and dimensioned to maintain the necessary wall thickness for increased fatigue value of the proximal conical flare 50. It will be appreciated that as the size of the stem component 11 increases, the angle of surface 56 may decrease to maintain the desired wall thickness. Likewise, as the size of the stem component 11 decreases, the angle of surface 56 may increase to maintain the desired wall thickness.

It will be appreciated that the femur comprises isoelastic properties, such that it will readily expand and contract. Accordingly, the proximal conical flare 50 may be configured to micro settle or micro subside into a position of stability as expansion and contraction of the femur occurs. As the proximal conical flare micro settles or subsides it will produce a compression load such that the proximal conical flare 50 may aid in transferring unnatural hoop stresses exerted on the device 10 into more natural compressive loads. It will further be appreciated that the conical features of the present invention, whether a conical proximal portion 14, or the rounded contour or rounded shape of the proximal conical flare 50, may provide a mechanism that may fit and fill the proximal cavity of the femur and that will not "hang up" on any portion of the cortical bone, or will not prematurely stabilize on a portion of the conical bone. Premature stabilization may result in aseptic loosening of the device 10, which may cause the device 10 to fail. Therefore, the conical features of the present invention may avoid aseptic loosening and provide for a device 10 that will not prematurely stabilize within the cavity of the bone by being hung up on the cortical bone. Accordingly, the conical proximal flare 50 may stabilize into a position of stability within the cavity.

It will be appreciated that the proximal conical flare 50 may further be comprised of a top surface 52 as illustrated. The proximal conical flare 50 may be tapered and have a symmetrical taper ratio per each side of the proximal conical flare 50. It will be appreciated that the taper ratio may be calculated by one of skill in the art having possession of this disclosure without undue experimentation.

As the stem component 11 micro subsides into its position of stability over time, it is possible that the entire stem 11 may settle several millimeters within the cavity. In such a case, the modular neck component 30 of the present invention advantageously permits a surgeon the opportunity to go back to the surgical site and replace one modular neck component 30 with another longer modular neck component 30 without interrupting the interface between the femur and the stem component 11, such that joint laxative and potential dislocation may be avoided. Therefore, the modularity of the neck component 30 allows for some potential correction in the hip joint of the device 10 with minimal disruption to the device 10.

It will be appreciated that in a natural femur stress is loaded from the outside in, whereas in a prosthetic femoral component stress is loaded from the inside out. One aspect of the device 10 of the present invention may be to transmit the forces to the outer, harder cortical bone as opposed to the inner, softer cancellous bone. The conical or bowl shaped contour of the proximal conical flare 50 of the present invention advantageously provides compressive loads, as opposed to hoop loads, and allows finite subsidence of the proximal conical flare 50 to a more stable position, as well as stabilizing the stem component 11 of the device 10 within the prepared medullary cavity. Therefore, as stresses are placed on the device 10, the proximal conical flare 50 may direct and transmit the forces to the outer cortical bone, such that the forces may be evenly distributed through the entire bone. As the proximal conical flare 50 subsides into the more stable position, the lateral offset of the device 10 may change. Advantageously, the modularity of the neck 30 allows for the adjustment of the lateral offset as described above by changing the length of the modular neck 30, thus restoring the lateral offset to more accurately simulate the biomechanics of the natural femur.

As mentioned previously, the proximal portion 14 may also include the anterior metaphyseal tapering flare 80 (illustrated best in FIGS. 4, 5 and 10) that may be configured to correspond with and even match the anatomical shape of the proximal femur and the metaphyseal cavity. As illustrated in FIGS. 4 and 5, the anterior metaphyseal tapering flare 80 may be located anteriorly on the proximal portion 14 of the stem component 11. The proximal portion 14 of the stem component 11 may be defined as having an anterior surface area, represented by the bracket 15, that may defined by a plane passing through the longitudinal axis A-A and that is perpendicular to the plane of the page. The proximal portion 14 may further be defined as having a posterior surface area, represented by the bracket 17, that may defined by a plane passing through the longitudinal axis A-A and that is perpendicular to the plane of the page. When the anterior metaphyseal tapering flare 80 is present, the anterior surface area of the proximal portion 14 may be greater than the posterior surface area of the proximal portion 14. The anterior metaphyseal tapering flare 80 may provide solid contact with an anterior portion of cortical bone thereby transferring stress from the device 10 to the bone.

The anterior metaphyseal tapering flare 80 may also comprise an enlarged portion 81 that protrudes from the anterior side 18 of the proximal portion 14, and configured as an anatomical body to engage the cortical bone to thereby transfer stress from the device to the bone. The anterior metaphyseal tapering flare 80 may further comprise a surface 82. The surface 82 may taper at an angle relative to a line D-D parallel to the longitudinal axis A-A, designated as α, the taper angle α being within a range of about ten degrees to about twenty degrees. For example, applicants have found a taper angle α of about twelve to about eighteen degrees to be a useful taper angle for the surface 82, and more specifically a range of about fourteen degrees to about sixteen degrees. In addition to the above range of angles for surface 82, the taper angle α may, for example, be about ten degrees, or about twelve degrees, or about fourteen degrees, or about sixteen degrees, or about eighteen degrees, or about twenty degrees.

It will be appreciated that the surface 82 may begin tapering, at the taper angle α listed above, from the proximal end 11a of the stem component 11 distally toward the distal end 11b of the stem component 11 for approximately one-half the length of the entire proximal portion 14 of the stem component 11. It will be appreciated that the length of the surface 82 may be modified to be greater than or less than one-half the length of the proximal portion 14,

As illustrated best in FIG. 5, the surface 82 and the remaining proximal portion 14 of the stem component 11 may meet at a location or junction, designated generally by 13, and thereafter the outer surface of the proximal portion 14 may continue to taper at an angle relative to the axis D-D, designated as β. It will be appreciated that both the anterior and posterior sides 18 and 19 may taper at the angle β, and the taper angle β of the remaining proximal portion 14 and distal portion 16 of the stem component 11 may be between the range of about three degrees to about six degrees per side. For example, applicants have found a taper angle of about four degrees per side to be an adequate taper angle. It will be appreciated that the taper angle β may be increased or decreased such that the taper occurs at a greater or lesser angle. It will likewise be appreciated that the surface 82 may straighten out at the location, designated by 13, such that no taper remains in the distal portion 16, and the distal portion 16 may instead comprise a uniform cross section.

It will be appreciated that the anterior metaphyseal tapering flare 80 may be configured for contacting and filling, at least a portion of, the proximal metaphyseal cavity of the proximal femur such that the anatomical features found on the proximal femur may be contacted by the anterior metaphyseal tapering flare 80. Thus, the anterior metaphyseal tapering flare 80 may contact at least a portion of the anterior cortex of the femur providing solid contact with the harder cortical bone to aid in distributing stresses placed on the device 10, and to increase resistance to torsional loads. It will be appreciated that the contact between the cortical bone and the anterior metaphyseal tapering flare 80 may also increase the stability of the entire device 10.

It should be noted that the anterior metaphyseal tapering flare 80 may be used in conjunction with the other aspects of the invention described herein, or the anterior metaphyseal tapering flare 80 may be used alone. For example, the anterior metaphyseal tapering flare 80 may be used in conjunction with the proximal conical flare 50 to provide maximum torsional load resistance and to provide increased intrinsic stability to the device 10. It will be appreciated that the anterior metaphyseal tapering flare 80 may be used in conjunction with any of the features of the present invention, and is not limited to being used with only the proximal conical flare 50.

The proximal portion 14 of the stem component 11 may also comprise a tapered exterior surface 75 (illustrated best in FIG. 2). The proximal portion 14 may be further characterized as being substantially conical with the anterior and posterior portions tapering toward the distal end 11b of a stem component 11 at an angle κ relative to a line F-F parallel to the longitudinal axis A-A, between a range of about three degrees to about six degrees per side. For example, applicants have found a taper angle of about four degrees per side to be an adequate taper angle. It will be appreciated that the taper angle may be increased or decreased such that the taper occurs at a greater or lesser angle. It will further be appreciated that the proximal portion 14 may comprise features, some of which have been described above such as the anterior metaphyseal tapering flare 80, that may change the taper of a part of the proximal portion 14, such that part of the proximal portion may either not taper, or taper at a greater or lesser angle than the tapered exterior surface 75. The tapered exterior surface 75 may be configured to provide surface contact with the proximal, cortical bone in the proximal femur. It will be appreciated that the taper and taper angle of the proximal portion 14 may be modified byone of skill in the art to include a greater or lesser taper, or taper angle, than illustrated in FIG. 2.

As mentioned previously, the tapered exterior surface 75 of the proximal portion 14, in one embodiment, may lead into a tapered exterior surface 76 of the distal portion 16 of the stem component 11 (illustrated best in FIG. 5). The tapered exterior surface 76 may continue at the same angle of taper as the tapered exterior surface 75 of the proximal portion 14, said taper angler β may be between the range of about three to about six degrees.

As illustrated in FIGS. 2 and 4, the distal portion 16 of the stem component 11 may comprise a rounded, distal tip 46. The distal tip 46 may have an opening located therein, which may correspond to an opening 61 of a coronal slot 60 that may be formed within the distal portion 16 of the stem component 11. The coronal slot 60 may be configured for allowing the distal portion 16 of the stem component 11 to bend as forces are exerted on the femur. It will be appreciated that the distal portion 16 of the stem component 11 may be shaped in any one of the following shapes, which distal portion 16 may be configured and dimensioned for implanting into the medullary canal of the femur to thereby anchor the prosthetic device 10: (i) a symmetrical straight distal stem having a substantially uniform cross section (illustrated in FIGS. 1-2, and 3-4); (ii) a tapered distal stem with a taper occurring on the exterior surface 76 of the distal stem (illustrated in FIGS. 5-8 and 10-15); or (iii) a curved stem. The curved stem, sometimes referred to herein as a bowed or an anatomical stem, may be used in situations where the bones are longer than average, and have need for a revision surgery.

As illustrated in FIGS. 2 and 4, the coronal slot 60, or any other slot that may be utilized by the present invention such as a helical slot 62 described more fully below, may extend longitudinally from approximately a mid portion 16a of the distal portion 16 down along the longitudinal axis A-A in a coronal plane, essentially separating the distal portion 16 of the stem component into an anterior portion 42 and a posterior portion 44. It will be appreciated that the length of the slot located within the distal portion 16, whether a coronal slot 60 or a helical slot 62, may comprise about twenty-five percent to about fifty percent of the entire length of the stem component 11. For example, applicants have found that a length of the slot that is about thirty-three percent of the entire length of the stem component 11 to be advantageous.

Additionally, the distal portion 16 of the stem component 11 may comprise at least one flute 43 for increasing torsional resistance. It will be appreciated that the at least one flute 43 may extend along the entire length of the distal portion 16, or the at least one flute 43 may extend along only part of the distal portion 16 without departing from the scope of the present invention. The at least one flute may be utilized to contact an inner surface of the medullary canal of the femur to thereby anchor the distal portion 16 of the stem component and to stabilize the device 10, thus resisting torsional forces that act on the femur.

It will be appreciated that one of the many challenges facing the surgeon in a hip replacement procedure is inhibiting what is referred to in the field as thigh pain. The everyday, repetitive movements that cause the leg to bend and twist introduce a substantial amount of stress in the femur, a large portion of which is transmitted through the inner core of the soft, cancellous bone, which has a larger degree of flexibility than the harder, cortical bone. It will be appreciated that if the stem component 11, and particularly the distal portion, is less flexible than the portion of the inner core of cancellous bone that it replaces, less stress will be distributed through the normal stress paths of the femur. Instead, the stress finds alternative, abnormal distribution paths though the thigh, thereby causing thigh pain.

The challenge in reducing thigh pain is heightened by the fact that the stem component 11 must have enough strength to withstand the normal torsional, bending and tension forces introduced thereto by the hip joint. Although materials have been developed in an attempt to accommodate all of these forces and stress transfers, the problem of thigh pain still remains. The coronal slot 60 was introduced to impart a limited degree of flexibility to the distal portion 16 of the stem component 11. As force is applied to the femur, the coronal slot 60 may allow the distal portion of the stem component 11 to compress somewhat to decrease some of the alternative stress distribution, thereby reducing thigh pain somewhat. Therefore, the coronal slot 60 may function to impart a limited degree of flexibility to the distal portion 16 of the stem component 11 and to the device 10 as a whole.

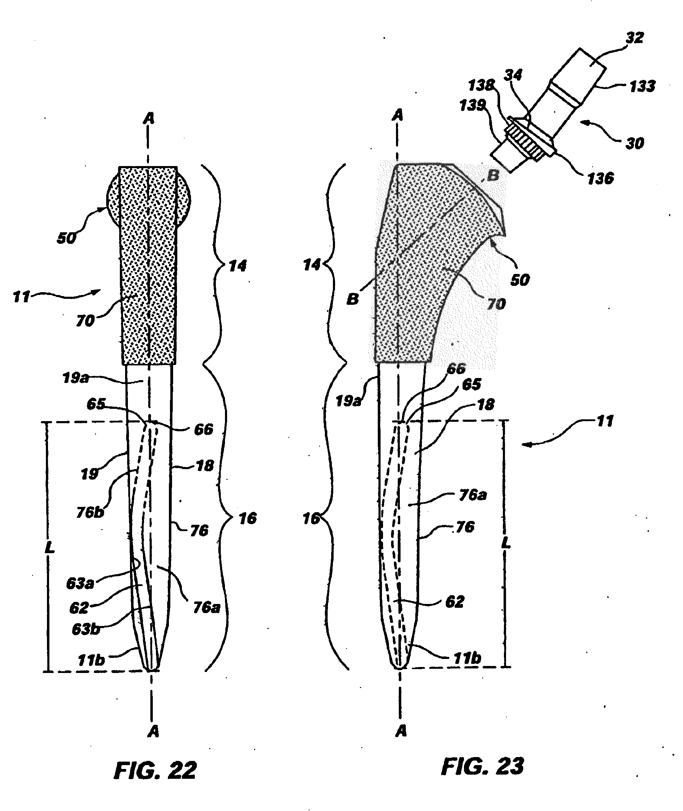

The coronal slot 60 is illustrated in FIGS. 2 and 4 as being straight and having no twists or curves in said slot 60. However, applicants have discovered that an alternative embodiment of the slot may further function to increase flexibility in the distal portion 16 of the stem component 11. FIGS. 22-23 illustrate the distal portion 16 of the stem component 11 as having the helical slot 62 referred to above. The helical slot 62 may comprise a longitudinal axis that may be the same as the longitudinal axis A-A of the stem component 11. The helical slot 62 may be defined by opposing inner walls 63a and 63b that may be substantially parallel to each other along a majority of a length "L" of the helical slot 62. It will be appreciated that the opposing inner walls 63a and 63b of the helical slot 62 may not be parallel near a proximal most portion 65 of the helical slot 62, where the opposing inner walls 63a and 63b may combine at a junction 66. The opposing inner walls 63a and 63b may twist within the exterior surface 76 of the distal portion 16 of the stem component 11 in a helical manner as illustrated, so as to essentially create two opposing forks 76a and 76b in the exterior surface 76 of the distal portion 16, wherein the two opposing forks 76a and 76b may also be twisted. It will be appreciated that the twisting of the slot 62 may extend at least partially around the exterior surface 76 and pass through the anterior side 18, the posterior side 19, and lateral side 19a of the distal portion 16. The twisting of the slot 62 may provide increased flexibility to the distal portion 16 of the stem component 11. The opposing inner walls 63a and 63b of the helical slot 62 may twist in such a manner so that the slot 62 may be visible by a human observer passing through three sides or surfaces of the stem component 11. It will be appreciated that the helical slot 62 may begin at the distal end 11b of the stem component 11 in the coronal plane. It is possible that the helical slot 62 may not complete a full twist, wherein a full twist may be defined as the inner walls 63a and 63b each making one complete rotation around the distal portion 16 of the stem component 11. The helical nature of the slot 62 allows the distal portion 16 to more closely simulate the physiological twisting and bending that occurs in the femur due to the torsional and bending forces that may be placed thereon. It will be appreciated that during normal daily activities, the human body may experience torsional forces that may be applied to the hip joint and to the femur, and the helical slot 62 of the stem component 11 may permit the stem component 11 to twist and compress somewhat in response to those torsional forces. Additionally, the helical slot 62 may permit the stem component to bend as a bending force is applied to the femur. Therefore, the helical slot 62 may impart more flexibility to the distal portion 16 of the stem component 11, than the coronal slot 60, or a sagittal slot 64, or even a V-slot (not illustrated in the FIGS.) individually. Accordingly, a limited degree of flexibility may be imparted to the distal portion 16 of the stem component 11. As force is applied and the helical slot 62 allows the distal portion 16 of the stem component 11 to compress somewhat, some of the alternative stress distribution may also be decreased, thereby reducing thigh pain. Therefore, the helical slot 62 may be advantageously used to reduce thigh pain due, at least in part, to the helical nature of the slot 62, which more closely simulates the ability of the natural femur to twist and bend.

Referring now to FIGS. 3 and 4, wherein an alternative embodiment of the present invention is illustrated as having similar components as the embodiment of FIGS. 1 and 2, with the exception of the anterior metaphyseal tapering flare 80, referred to above, which may also be provided. As previously discussed, the flare 80 may be configured on the anterior side 18 of the femoral prosthetic device 10 such that the flare 80 may aid in filling, at least in part, the metaphyseal cavity of the femur more completely, such that contact between the anterior metaphyseal tapering flare 80 and the cortical bone may occur. Thus, the flare 80 may be a mechanism for resisting the torsional loads that are commonly placed on the femoral prosthetic device 10. It should be noted that the anterior metaphyseal tapering flare 80 may be configured to be of any suitable size in order to create an area of contact between the hard, cortical bone of the anterior cortex of the proximal femur and the device 10. It will be appreciated that the size of the anterior metaphyseal tapering flare 80 may correspond to the size of the medullary cavity created at the top of the medullary canal and may therefore be of any suitable size to fill such an anatomical area. The anterior metaphyseal tapering flare 80, therefore, creates an area of contact with the cortical bone portion of the femur and functions to distribute loads from the device 10 to the bone and also to increase resistance to torsional loads.

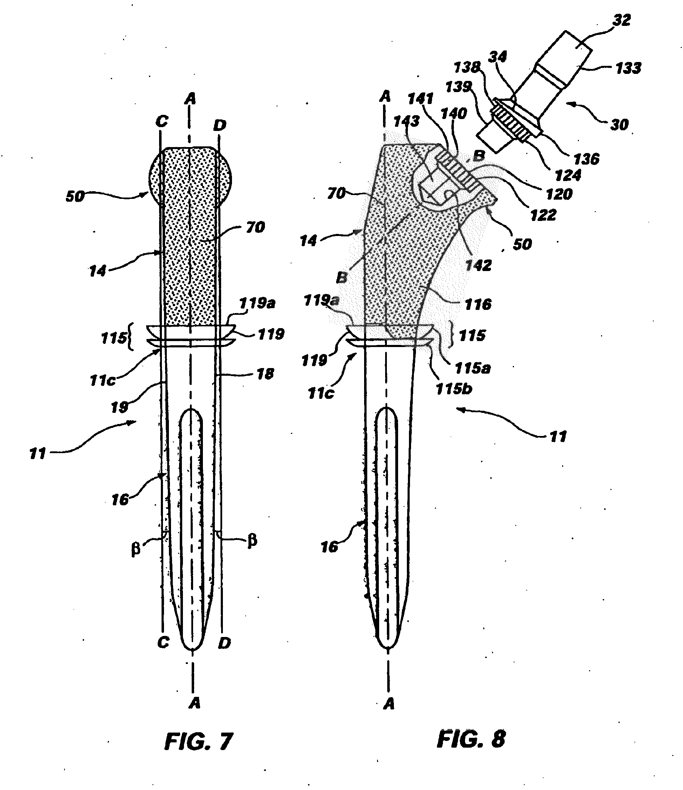

Referring now to FIGS. 7-8, it will be appreciated that during a revision surgery it may be difficult to remove the stem component 11 from the femur without removing or damaging valuable bone, especially when the stem component 11 has been cemented distally. FIGS. 7-8 illustrate a hybrid stem component 11 that may be implanted into the cavity or canal of the bone using bone cement or other biocompatible material for fixating the proximal portion 14 of the stem component 11 within the cavity or canal, while the distal portion 16 of the stem component 11 may be press-fit into the canal of the bone.

The stem component 11 may comprise, whether a hybrid stem or not, a rough surface 116 located on the proximal portion 14 of the stem component 11 for increasing the interdigitation between bone or bone cement and the proximal portion 14, to thereby increase the strength of the fixation. It will be appreciated that the rough surface 116 may be created using different materials depending upon the application, whether a cementless application is used or a hybrid cemented application is used. Examples of the materials that may be used to create the rough surface finish on the proximal portion 14 include matte, porous, HA, porous HA, combinations thereof, or beads, or other finishes.

In the hybrid cemented application, a coating of beads, for example 0.5 mm in size, that have been bead blasted onto the surface of the proximal portion 14 may be used to increase the surface area of the proximal portion 14, thereby increasing the interdigitation between the bone, the bone cement, and the proximal portion 14 of the stem component 11, such that a more secure proximal fixation of the stem component 11 to the bone may be achieved.

It should be noted that the roughness and method of applying the surficial roughness to the proximal portion 14 may be as described above, or the rough surface 116 may be corrugated or any other mechanism for producing a roughened surface to provide increased surface area. The method for manufacturing the surficial roughness may include any method presently known, or which may become known in the future, in the art for adding a surficial roughness to the proximal portion 14 of the stem component 11. Additionally, the material, design and shape used to create the roughness may be modified by one of skill in the art using any suitable material, design and shape presently known, or which may become known, in the art for increasing the surface area and interdigitation of the proximal portion 14 of the stem component 11. It will be appreciated that other components or parts of components may also have the rough surface finish, such as the neck component 30. Further, the area that the roughness comprises on the stem component 11 or neck component 30 may vary depending upon the desired outcome, which can be determined by one of skill in the art.

Additionally, FIGS. 7-8 illustrate a tapering proximal portion 14, wherein the anterior side 18 and the posterior side 19 both slope at the angle β, the modular neck component 30, and the recess 120. It will be appreciated that the modular neck component 30 and the recess 120 as illustrated in each embodiment of the present invention may comprise the modular features as described above in connection with the modular neck component 30.

In the hybrid stem component 11 of FIGS. 7-9, the proximal portion 14 may be separated from the distal portion 16 by a restrictor 115, that may also act as a centralizer. The restrictor 115 may be manufactured from a resilient material such as a thermoplastic, for example silicone, polyethylene, or polypropylene, or the restrictor 115 may be manufactured from a metal that does not exhibit the same resilient characteristics as thermoplastics, or the restrictor 115 may be made from bone. The restrictor 115 may at least partially surround the stem component 11, and may be slightly bowl shaped. The restrictor 115 may have an exterior surface 119 and a depression 119a formed therein giving the restrictor its bowl shape. Additionally, the restrictor 115 may comprise two lobes, a first lobe 115a and a second lobe 115b, with the firs lobe 115a residing above the second lobe 115b. The restrictor 115 may be positioned in engagement with the stem component 11 in an upward attitude, essentially separating the proximal portion 14 from the distal portion 16 near a mid-stem 11c. The restrictor 115 may function to keep a substantial amount of bone cement from entering into the area of the cavity or canal, which is located distally to the position of the proximal portion 14 when the stem component 11 is located within the cavity or canal of the bone.

In the hybrid stem component 11 of the present invention, the basic concept may comprise a custom fit and fill in the proximal portion 14 of the stem component 11 in the proximal metaphyseal cavity of the femur, such that the proximal portion 14 of the stem component 11 and the bone cement may fill the variable proximal metaphyseal shape of the proximal femur. Conversely, the distal portion 16 of the stem component 11 may be press-fit, and not cemented, into the distal portion of the cavity or canal in the proximal femur such that the stem component 11 may be removed during a revision surgery with minimal bone disruption distally, should removal become necessary.

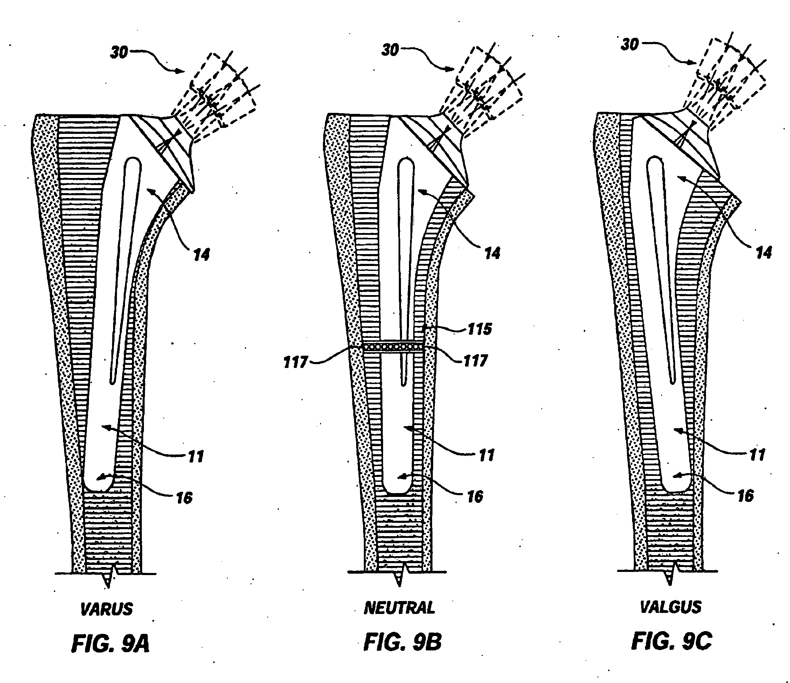

Referring now to FIGS. 9A-9C, in the orthopedic industry after the stem component 11 has been implanted within the metaphyseal cavity of the femur, it has become a relatively common occurrence for the stem component 11 to become malaligned within the cavity over time. If the neck component 30 and the stem component 11 slip downward causing the distal portion 16 to move farther laterally, the stem component 11 may be said to have slipped into a varus position, as illustrated by FIG. 9A. Conversely, if the neck component 30 and the stem component 11 move upward causing the distal portion 16 to move farther medially, the stem component 11 may be said to have moved into a valgus position, as illustrated in FIG. 9C.

As illustrated in FIG. 9B, the restrictor 115 may also function as the centralizer referred to above to maintain the stem component 11 in a proper, centralized orientation within the metaphyseal cavity. The restrictor 115 may be dimensioned such that an outer surface 117 of the restrictor 115 may contact the inner wall of the metaphyseal cavity forming a friction fit between the restrictor 115 and the inner wall of the cavity, thus stabilizing the stem component 11. It will be appreciated that the restrictor 115 may surround the stem component 11, and may be further characterized as a rounded sleeve. It will be appreciated that the restrictor 115 may be utilized as a cement restrictor only, as a centralizer only, or as both a cement restrictor and as a centralizer,

Practically, the process of implanting the hybrid stem component 11 may include the following. First, insert the stem component 11 about half-way into the metaphyseal cavity so that the distal portion 16 sits essentially within the metaphyseal cavity with the top of the restrictor 115 being readily accessible. Second, add a viscous bone cement to the metaphyseal cavity to fill the cavity. Last, continue to insert the stem component 11 into the cavity until the proximal portion 14 of the stem component 11 may be securely seated therein. Thus, the proximal portion 14 may be seated within the cavity and surrounded by bone cement, whereas the distal portion 16 may be press-fit into the cavity securing the stem component 11 to the bone.

Regarding the hybrid stem component 11, applicants have found that the stem component 11 manufactured from cobalt-chromium alloy material, because of its stiffness, will not put the same amount of stress on the interface between the stem component 11 and the cement mantle as a titanium alloy stem component 11. Accordingly, the hybrid stem component 11 utilizes the advantages of cobalt-chromium alloy, which is the material of choice in cemented applications, to interface with the bone cement on the proximal portion 14 to thereby reduce the stress placed on the cement mantle interface. Accordingly, the hybrid stem component 11 may be manufactured from cobalt-chromium alloy to increase the chances of clinical success.

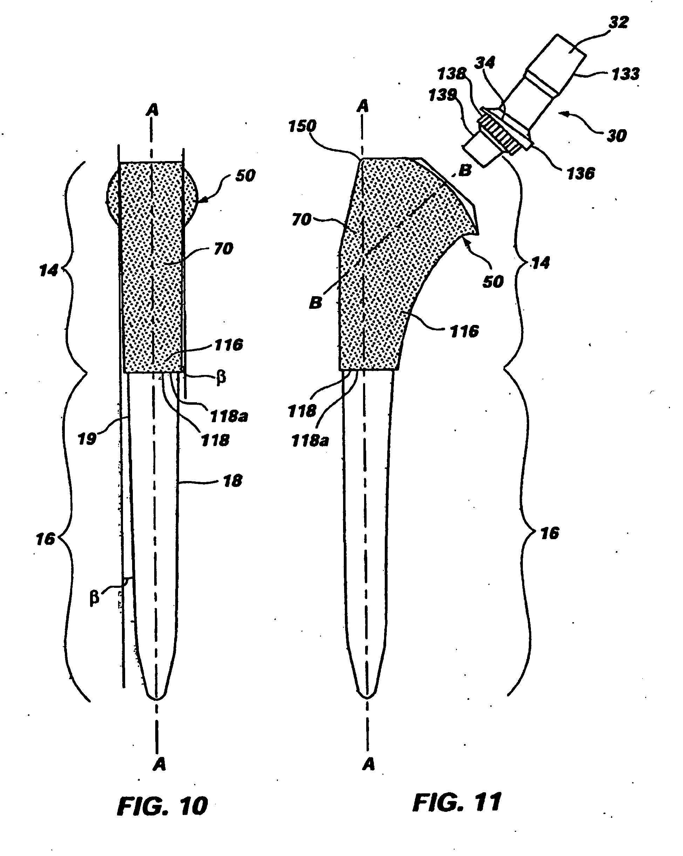

Referring now to FIGS. 10-11, the stem component 11 is illustrated as being collarless and is further illustrated in conjunction with the modular neck component 30. It will be appreciated that the embodiment of the invention illustrated in FIGS. 10-11 may contain many of the same features and/or structures represented in previous FIGS., and only the new or different features and structures will be explained to most succinctly explain the additional advantages which come with the embodiment of the invention illustrated in FIGS. 10-11. The proximal portion 14 of the stem component 11, as illustrated, may comprise a taper that may be similar to the taper of the distal portion 16. As illustrated, both the proximal portion 14 and the distal portion may taper on both the anterior and posterior sides 18 and 19 at the taper angle β, and the taper angle β may be between the range of about three degrees to about six degrees per side. For example, applicants have found a taper angle of about four degrees per side to be an adequate taper angle. It will be appreciated that the proximal portion 14 may be separated from the distal portion 16 by a junction 118a that may form a lip. It will be appreciated that the lip 118 may or may not be present, but when the lip 118 is present, it may be round and smooth so as to avoid creating stress risers at that junction.

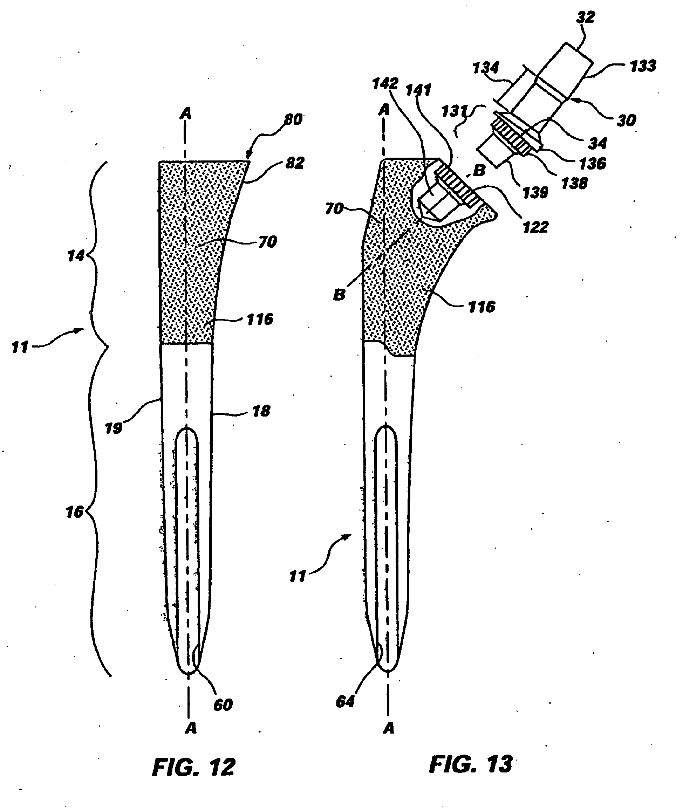

Referring now to FIGS. 12-13, the stem component 11 is illustrated with the anterior metaphyseal tapering flare 80. It will be appreciated that the embodiment of the invention illustrated in FIGS. 12-13 may contain many of the same features and/or structures represented in previous FIGS., and only the new or different features and structures will be explained to most succinctly explain the additional advantages which come with the embodiment of the invention illustrated in FIGS. 12-13. As illustrated, the distal portion 16 may comprise the coronal slot 60, in addition to a sagittal slot 64. The addition of the sagittal slot 64 may permit additional bending and compression of the distal portion 16 of the stem component 11 as forces are placed on the femur and the device 10. It will be appreciated that the helical slot 62 may also be utilized in this embodiment. No matter which slot, or combination of slots, is used the slot may comprise about twenty percent to about sixty percent of the stem component 11, and may be formed within the distal portion 16 beginning at the distal end 11b of the stem component 11 and extend proximally toward the proximal end 11a. For example, applicants have found that a slot that comprises about thirty-three percent to about fifty percent of the stem component 11 to be useful. Another useful example may comprise about thirty-three percent to about forty percent of the stem component 11.

It will be appreciated that the type of material used to manufacture the device 10 as a whole, and each of the component parts may affect the interface between the device 10 and the bone, or bone cement in some embodiments. Accordingly, several different materials may be utilized by the present invention, including metal, such as titanium, stainless steel, cobalt-chromium-molybdenum alloy, titanium-aluminum vanadium alloy, or other alloys thereof. It will further be appreciated that the properties of various metals differ with respect to their relative hardness, tensile strength, and yield strength. For example, according to ASTM designation: F136-98, forged titanium-6aluminum-4vanadium alloy has a tensile 0,861KN/mm² strength of (125.000 psi) and a minimum yield strength of 0,793KN/mm² (115.000 psi) (hereinafter referred to as "forged titanium"). While forged cobalt-28chromium-6molybdenum alloy has a tensile strength of 1,172KN/mm² (170.000 psi), and a yield strength of 0,827KN/mm² (120.000 psi), according to ASTM designation: F799-99 (hereinafter referred to as "forged cobalt-chromium"). Additionally, cast cobalt-28chromium-6molybdenum alloy has a tensile strength of 0,655/KNmm² (95.000 psi) and a yield strength of 0,448KN/mm² (65.000 psi), according to ASTM designation: F75-98.

It will be appreciated that one of the many factors in choosing a material to design an artificial hip device is the tendency for the device to corrode, particularly at modular taper fitting sites, where crevice corrosion may occur. According to an article by M. Viceconti et al., "Design-related fretting wear in modular neck hip prosthesis," Journal of Biomedical Materials Research, Vol. 30, 181-166 (1996), traditionally, forged titanium has been used in the industry to combat the results of corrosion with relative success. The success of forged titanium is due, at least in part, to the very thin layer of titanium oxide that covers the whole surface of the implant, under normal conditions. The titanium oxide layer's chemical properties protects the forged titanium even in very harsh conditions, such as those found in a human body. However, even with forged titanium, modular sites and taper fitting sites may be subject to corrosion due to: (1) the abrasion of the forged titanium causing damage to the protective layer causing fretting corrosion, and (2) the small volumes of fluid that may be trapped causing crevice corrosion.

Additionally, "notch sensitivity" may also induce undesirable corrosion and cracking, as the minor nicks, and cracks in the implant may induce further corrosion, cracking and wear as the harsh conditions of the human body act on the implant. As modular forged titanium prostheses have become standard in the orthopedic industry, the occurrence of corrosion of forged titanium implants has increased. Accordingly, to minimize or reduce corrosion, applicants have used forged cobalt-chromium, which stress shields the bone more effectively than forged titanium due to its stiffer properties, in prosthetic components, including modular neck components 30 and stem components 11, to aid in the reduction of corrosion and other problems associated with modular junctions using forged titanium.

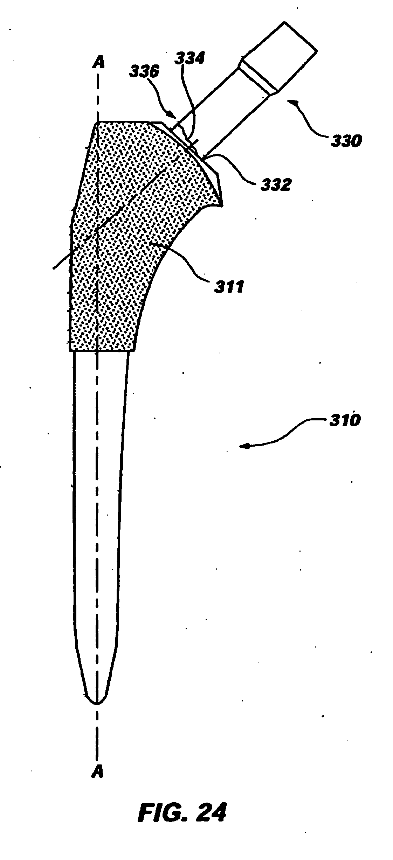

FIG. 24 illustrates a failed forged titanium alloy femoral prosthetic device 310. The forged titanium alloy device 310 may be damaged from forces acting on the device 310 in the human body. As illustrated, the forged titanium alloy has become damaged to the point of failure, due to the harsh environment of the human body and specifically in the hip joint and also due to the fatigue properties and fatigue potential of forged titanium alloy. Accordingly, FIG. 24 illustrates the neck component 330 having a fracture 334 at its base 332. The fracture 334 started on a superior-lateral side 336 of the neck component 330 and has extended through approximately two-thirds of the neck component 330. While not illustrated in FIG. 24, it is possible for the fracture 334 to extend completely through the entire neck component 330, essentially severing the neck component 330 from the stem component 311.

Forged cobalt-chromium is a metal that has a higher tensile strength and higher yield than forged titanium. As such, forged cobalt-chromium is stiffer than forged titanium, and therefore absorbs more load and is able to distribute the stress placed on the device 10 over a larger area than forged titanium. Accordingly, the device 10, made of forged cobalt-chromium, may not impose as much stress on the cement implant interface than a device 10 made of forged titanium thereby reducing aseptic loosening of the stem.

However, it has been demonstrated that forged titanium has significant biocompatible properties that permits bone to grow around and even into the forged titanium. Accordingly, forged titanium has been used extensively in the orthopedic industry not only for cementless stem applications, but also in cemented stem applications.

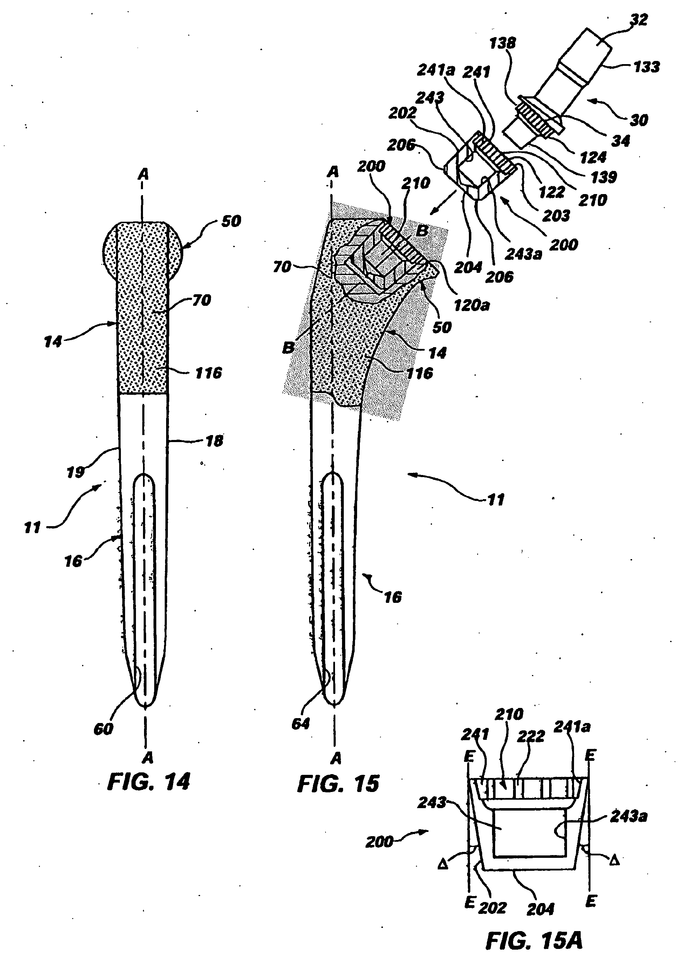

Reference will now to made to FIGS. 14-19 to describe another embodiment of the modular neck component 30 and its attachment to the stem component 11. It will be appreciated that the embodiments of the invention illustrated in FIGS. 14-19 may contain many of the same features and/or structures represented in previous FIGS., and only the new or different features and structures will be explained to most succinctly explain the additional advantages which come with the embodiments of the invention illustrated in FIGS. 14-19.

As illustrated in FIGS. 14-19, the device 10 may further comprise a bushing insert 200, sometimes referred to as a sleeve, which may be configured and dimensioned to correspond with the recess 120, such that the busing insert 200 may fit into said recess 120. FIGS. 15, 17, and 19 illustrate the bushing insert 200 as being inserted and assembled into the recess 120, and also illustrate the bushing insert 200 in an exploded view.

It will be appreciated that the busing insert 200 may comprise the structural features present in the recess 120 as described in connection with earlier embodiments, leaving the recess 120 essentially free of those components. For example, the bushing insert 200 may comprise its own recess 210, which may comprise a first portion 241 defined by a first sidewall 241a, and a second portion 243 defined by a second sidewall 243a, which are similar to the first portion 141 and the second portion 143 of the recess 120. Accordingly, the first portion 241 may include a corresponding second splines 222 for matingly engaging the first splines 124 of the outer tapered portion 138 of the modular neck component 30 so that the modular neck component 30 may be indexed within the bushing insert 200, which indexing is described more fully above in connection with FIGS. 1A-1D. Additionally, the bushing insert 200 may comprise an outer wall 202, a top surface 203, a bottom surface 204, and may also comprise chamfered edges 206. The chamfered edges 206 permit the bushing insert 200 to easily enter into the recess 120 without interference from the structure surrounding the recess 120.

It will be appreciated that the bushing insert 200 and the recess 120 may both be shaped similarly. In each of the embodiments containing the bushing insert 200 and the recess 120, the shape of the bushing insert 200 and recess 120 may be any suitable shape known in the art. For example, the bushing insert 200 and corresponding recess 120 may be circular or oval; or triangular, square, hexagonal or any other polygonal shape, which may be utilized as the shape for the bushing insert 200 and recess 120.

The bushing insert 200 may be configured and dimensioned to seat within the recess 120, and the bushing insert 200 may be attached to the recess 120 by any one of the following locking mechanisms: (1) a taper lock, or taper press-fit; (2) a mechanical interlock; or (3) a press-fit lock.

Referring particularly to FIGS. 14-15, the taper lock may occur between the outer wall 202 of the bushing insert 200 and an inner sidewall 120a of the recess 120. Referring specifically to FIG. 15A, the outer wall 202 of the bushing insert 200 may surround the opening into the first portion 241 and second portion 243, and may be tapered at an angle Δ relative to a line E-E parallel to a long axis of the bushing insert, wherein the taper may fall within the range of angles that are of the self-locking type. The inner sidewall 120a of the recess 120 may also be tapered at a taper angle that corresponds to the taper angle Δ, such that a self-locking connection between the outer wall 202 of the bushing insert 200 and the inner sidewall 120a of the recess 120 may occur. Specifically, engagement between the outer wall 202 and the inner sidewall 120a may occur forming the taper fit, locking the bushing insert 200 to the recess 120. Thus, the bushing insert 200 may be secured and locked within the recess 120 via the self-locking taper.

Additionally, the taper angle Δ of the outer wall 202 and the inner sidewall 120a may taper at an angle between a range of about one degree to about three degrees per side for forming a taper press-fit. For example, the taper angle Δ may be between one and two degrees. The outer wall 202 and the inner sidewall 120a may matingly engage one another by way of a taper press-fit, wherein the bushing insert 200 may be slightly larger than the recess 120. Accordingly, the outer wall 202 may contact the inner sidewall 120a creating an intimate taper press-fit.

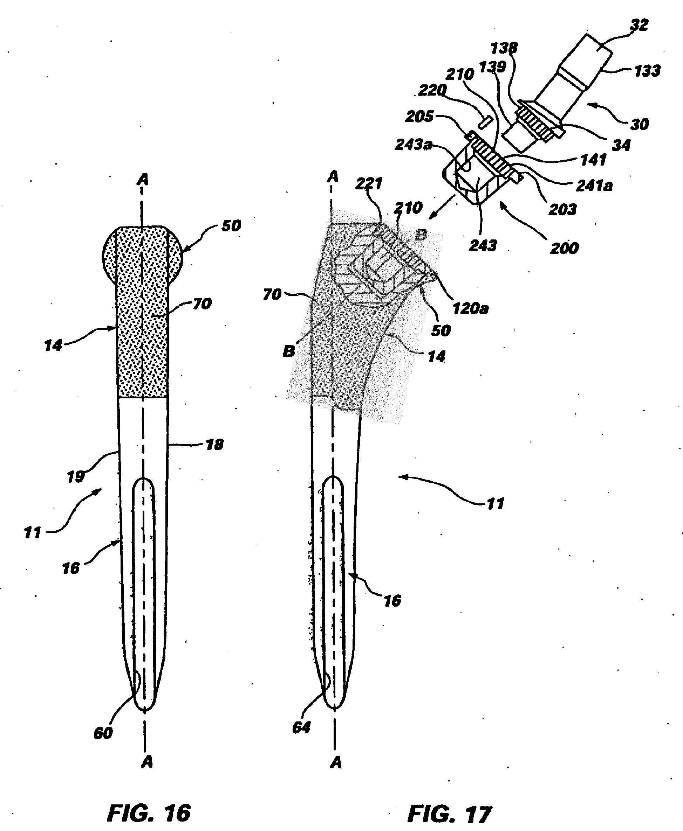

Referring now to FIGS. 16-17, the bushing insert 200 may be locked to the recess 120 by using the mechanical interlock referred to above. The bushing insert 200 may comprise a keyway 205 formed in the top surface 203, which may be configured to receive a key 220, also referred to as a pin or bayonet. The keyway 205 may be formed as a through hole such that the key 220 may pass therethrough and fit into a corresponding notch 221 in the proximal portion 16 of the stem component 11 near the entrance of the recess 120. It will be appreciated that the key 220 may be dimensioned to fit or wedge within the notch 221 to thereby form a lock, locking the bushing insert 200 within the recess 120 and to the proximal portion 14 of the stem component 11.

It will be appreciated that the key 220, keyway 205, and notch 221 may all be modified to include various shapes and designs known to those of ordinary skill in the art for forming a mechanical interlock between two components. Additionally, it will be appreciated that other mechanical interlocks may be utilized. For example, the bushing insert 200 may be mechanically interlocked with the recess 120 by twisting the bushing insert 200 a quarter twist within the recess 120 mechanically engaging portions from the bushing insert 200 and recess 120 forming an interference fit.

Referring now to FIGS. 18-19, the bushing insert 200 may be locked within the recess 120 via the press-fit lock referred to above. In this embodiment, the recess 120 may have a first portion 141a and a second portion 143a (illustrated best in FIG. 19A), or the recess 120 may comprise only the first portion 141a comprising the inner sidewall 120a (illustrated best in FIG. 19). FIG. 19A illustrates the embodiment of the bushing insert 200 that may comprise the outer wall 202 and may further comprise an upper wall surface 202a disposed above the outer wall 202. FIG. 19A also illustrates the corresponding recess 120 for the bushing insert 200 of FIG. 19A. The second portion 143a of the recess 120 may be defined by the inner sidewall 120a, also referred to herein as a first inner sidewall 120a of the recess 120, and the first portion 141a of the recess 120 may be defined by a second inner sidewall 120b. It will be appreciated that the outer wall 202 and the upper wall surface 202a, and the first inner sidewall 120a and the second inner sidewall 120b may be cylindrically shaped. It will be appreciated that the inner sidewall 120a of the recess 120 and the outer wall 202 in FIG. 19 may also be cylindrically shaped.

It will be appreciated that the outer wall 202 and the upper wall surface 202a of the bushing insert 200 of FIGS. 19 and 19A may be slightly larger than the first inner sidewall 120a and the second inner sidewall 120b of the recess 120 such that the outer wall 202 and the upper wall surface 202a may bite slightly into the first inner sidewall 120a and second inner sidewall 120b, respectively, forming a friction press-fit lock as the bushing insert 200 is pressed into the recess 120 under force. It is to be understood that the friction press-fit lock of FIG. 19 may also be formed as described above in connection with FIG. 19A, but may only be formed between the outer wall 202 and inner sidewall 120a.

It will be appreciated that the friction press-fit and associated contact between surfaces may occur along a majority of those surfaces, forming a very strong connection. Thus, the press-fit may occur between two corresponding surfaces, namely between: (1) the upper wall surface 202a and the second inner sidewall 120b, and (2) the outer wall 202 and the first inner sidewall 120a. It will be appreciated that the press-fit lock designed to lock the bushing insert 200 to the recess 120 may also be formed between only one of the corresponding surfaces listed above (either (1) or (2)), and a press-fit occurring in two separate locations is not required. Accordingly, either press-fit taken alone may function to lock the bushing insert 200 to the recess 120.

Applicants have conceived of a device 10 that may minimize the problems associated with forged titanium at the modular junctions, i.e. between the neck component 30 and the recess 120 in the stem component 11, by taking advantage of the mechanical properties of both forged titanium and forged cobalt-chromium. It will be appreciated that the head component, the neck component 30, the stem component 11, and the bushing insert 200 may each be manufactured from either forged cobalt-chromium, cast cobalt-chromium, or forged titanium, or any combination thereof without departing from the scope of the present invention. However, applicants have discovered that loads placed on the neck/stem junction may be effectively distributed and the results of fatigue, and problems associated with the fatigue of forged titanium and cast cobalt-chromium, may be minimized by using a stem component 11 manufactured from either forged titanium or cast cobalt-chromium, and a modular neck component 30 and bushing insert 200 manufactured from forged cobalt-chromium.

It will be appreciated that because of the forged cobalt-chromium material, the forces acting on the modular neck component 30 may be effectively and evenly distributed to the bushing insert 200. The bushing insert 200, having a greater surface area than the neck component 30, may further distribute the forces through the forged titanium stem component 11. The stem component 11 comprises a large surface area and thereby distributes the remaining stress through to the bone. Therefore, the bushing insert 200 may protect the forged titanium stem component 11 at the junction of the stem/neck from stress, such that the forged titanium will not encounter the same level of stress. Accordingly, the forged titanium stem component 11 may be subject to less force, such that there is less of a chance the stem component 11 will experience damage.

The forged cobalt-chromium bushing insert 200 may also reinforce the junction between the neck component 30 and the recess 120 of the stem component 11 such that there is a junction comprising forged cobalt-chromium on forged cobalt-chromium, which is a stronger connection than an all forged titanium connection. Therefore, the bushing insert 200 may effectively act as a fatigue reinforcer and as a load distributor to protect the stem component 11 from damage.

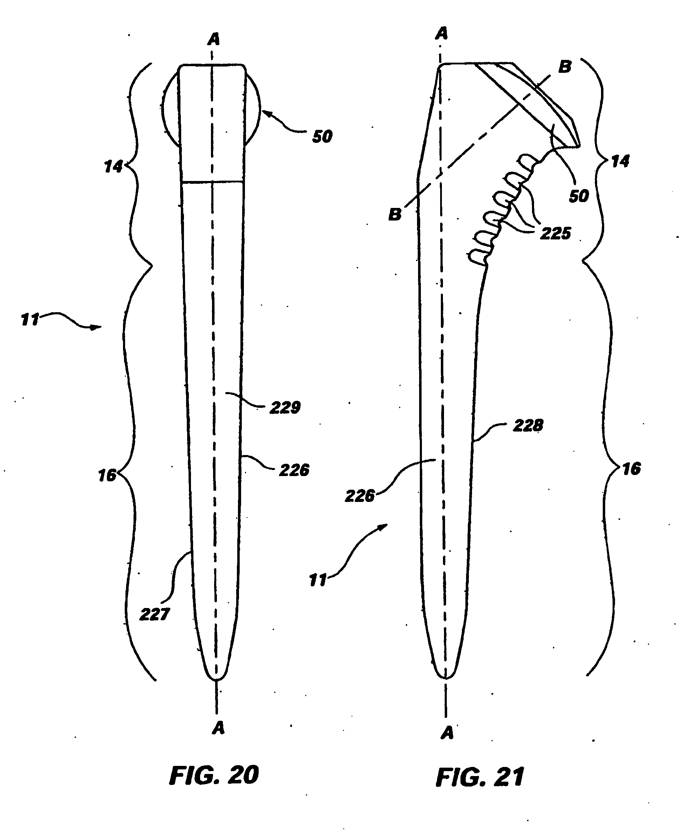

Referring now to FIGS. 20-21, the stem component 11 is illustrated as being collarless for use as a fit and fill cementless stem. It will be appreciated that the example illustrated in FIGS. 20-21 may contain many of the same features and/or structures represented in previous FIGS., and only the new or different features and structures will be explained to most succinctly explain the additional advantages which come with the example illustrated in FIGS. 20-21. As illustrated, the stem component 11 may comprise a flat anterior surface 226, a flat posterior surface 227, a flat medial surface 228 and a flat lateral surface 229, wherein each of the surfaces 226-229 may taper at a slight angle with respect to the longitudinal axis A-A of the stem component 11. Accordingly, the stem component 11 may be substantially shaped as a wedge.

As illustrated in FIG. 21, the proximal portion 14 may comprise a series of depressions 225 formed on the medial side of the stem component 11. The depressions are configured and dimensioned to contact the medial portion of the bone such that bone ingrowth may be stimulated.

It should be noted that each of the above-described components may be used in conjunction with one another or in a combination with other specific features to create a device 10 that may be specifically tailored to the anatomical needs of each patient. For example, referring to FIGS. 5 and 6, the following features may be used in combination with one another: (i) the proximal conical flare 50; (ii) the anteverted modular neck 30; (iii) the anterior metaphyseal tapering flare 80; and (iv) the coronal slot 60. It should be noted, however, that one of skill in the art may modify the device to include more or fewer features in the overall femoral prosthetic device 10 than has been illustrated in FIGS. 5 and 6. For example, it will be appreciated that an integral neck 30 may be used in place of the modular neck 30, or a twisted or helical slot 62 may be used in place of a coronal slot 60.

In accordance with the features and combinations described above, a useful method of implanting a femoral prosthetic implant into a patient's hip joint by a surgeon includes the steps of:
(a); reaming a hole in a femur to expose the medullary canal of said femur;
(b) ascertaining the anatomy of the patient;
(c) determining the combination of intrinsic features to be used to simulate the anatomy of the femur and to resist torsional loads increasing the intrinsic stability of the device, including the following features: (i) a modular, indexable neck; (ii) an appropriate angle of anteversion; (iii) a proximal conical flare having a rounded bottom contour; (iv) an anterior metaphyseal tapering flare; (v) a straight stem; (vi) a curved stem; (vii) a straight coronal slot; and (viii) a helical slot;
(d) selecting an appropriate device having the appropriate combination of features; and
(e) implanting said device into the medullary canal. In accordance with the features and combinations described above, another useful method of implanting a femoral prosthetic implant into a patient's hip joint includes the steps of:
   (a) exposing an opening in a patient's medullary canal of a femur;
   (b) selecting a device having a combination of intrinsic stabilizing features including: (i) a modular, indexable neck; (ii) an appropriate angle of anteversion; (iii) a proximal conical flare having a rounded bottom contour; (iv) an anterior metaphyseal tapering flare; (v) a straight stem; (vi) a curved stem; (vii) a straight coronal slot; and (viii) a helical slot, said device further having a head portion, a proximal portion and a stem component; and
   (c) positioning the stem component within the medullary canal such that the proximal portion substantially fills the opening of the medullary canal.

Those having ordinary skill in the relevant art will appreciate the advantages provide by the features of the present invention. For example, it is a potential feature of the present invention to provide a femoral prosthetic device which is simple in design and manufacture. Another potential feature of the present invention is to provide such a femoral prosthetic device that is capable of increasing the resistance to the torsional loads that are placed upon the prosthetic device in the femur. It is another potential feature to provide optimum solid contact with the anterior cortical bone, while at the same time substantially filling the metaphyseal area of the femur. It is a further potential feature of the present invention to provide solid cortical contact in the femur without removing cortical bone in the posterior wall region of the femur.

A bushing insert may be located within the recess of the stem component, thereby acting as a stress distributor and a fatigue reinforcer. Another potential feature of the present invention is to provide a modular neck component having indexable capability and that further provides a double taper lock. It a potential feature to provide a stem component having one or more of the following features: a proximal conical flare, an anterior metaphyseal tapering flare, a coronal slot, a sagittal slot, a helical slot, a tapering distal stem portion, a straight distal stem portion, and a curved distal stem portion.

It is to be understood that the above-described arrangements are only illustrative of the application of the principles of the present invention. Numerous modifications and alternative arrangements may be devised by those skilled in the art without departing from the scope of the present invention as defined by the claims. Thus, while the present invention has been shown in the drawings and described above with particularity and detail, it will be apparent to those of ordinary skill in the art that numerous modifications, including, but not limited to, variations in size, materials, shape, form, function and manner of operation, assembly and use may be made without departing from the invention.

## Claims

1. A prosthetic device (10) for implantation into a bone, the device comprising:
a modular neck component (12) comprising a proximal end (32), a distal end (34), and an outer tapered portion (138), extending below the distal end of the modular neck component; and
a stem component (11) having a recess (120) formed in a proximal portion (14) thereof, said recess having a first portion defined by a first tapered sidewall (140), and a second portion defined by a second tapered sidewall (142); wherein the proximal portion (14) of said stem component (11) flares outwardly in a distal-to-proximal direction into an outward flare to thereby form a flared surface portion that forms a flare angle relative to a longitudinal axis of the stem component within a range of about fifteen to about forty-five degrees
**characterized in that** the modular neck component comprises an inner tapered portion (139) extending distally below the outer tapered portion (138), and **in that** the outer tapered portion (138), of the modular neck component (12) comprises a plurality of first splines (124), defined within a perimeter of the outer tapered portion, and wherein the first sidewall (140) of the first portion of the recess (120) comprises a plurality of corresponding second splines (122) for matingly engaging the plurality of first splines (124) of said modular neck component (12) to thereby position the modular neck component in multiple, predetermined orientations within said recess (120) of the proximal portion (14) of the stem component (11).

2. The prosthetic device of claim 1, wherein the outward flare of the proximal portion (14) of the stem component is a proximal conical flare (50) having an undersurface (54) with a contour that is shaped in a rounded conical manner such that the proximal conical flare fills, at least a portion, of a proximal metaphyseal cavity in the bone, wherein said proximal conical flare is configured to cause compression loading on the bone such that the proximal conical flare (50) aids in transferring unnatural hoop stresses exerted on the device (10) into more natural compressive loads;

3. The prosthetic device of claim 1 or 2, wherein the flare angle is selected from about eighteen degrees, about twenty degrees, about twenty-two degrees, about twenty-four degrees, about twenty-six degrees, about twenty-eight degrees, about thirty degrees, about thirty-two degrees, about thirty-four degrees, about thirty-six degrees, about thirty-eight degrees, about forty degrees, about forty-two degrees, or about forty-four degrees.

4. The prosthetic device of any preceding claim, wherein the stem component (11) has a longitudinal axis that is centered with respect to the distal portion of the stem component, wherein a plane runs through the longitudinal axis and separates an anterior side (18) and a posterior side (19) of said stem component (11), and wherein the proximal conical flare (50) further comprises a surface (56) that tapers forming a posterior flare (57) that is located proximally on the posterior side (19) of the stem component (11).

5. The prosthetic device of claim 4, wherein the surface is non-convex, and preferably the non-convex surface tapers without also extending through an ascending and descending range of varying angles.

6. The prosthetic device of claims 2 to 5 , wherein the proximal conical flare (50) further comprises a posterior radius (250) defined as a distance between a point (251) that is central with respect to the recess (120) of the stem component and an end (250a) of the proximal conical flare located on the posterior edge of the proximal conical flare such that the posterior radius is larger than an anterior radius.

7. The prosthetic device of claims 2 to 6, wherein the proximal conical flare (50) is formed in an upper most part of the proximal portion (14) of the stem component (11) and comprises about one percent to about twenty percent of an entire length of the stem component, preferably about four percent to about ten percent of the entire length of the stem component, and further preferably about six percent of the entire length of the stem component.

8. The prosthetic device of any preceding claim wherein the outer tapered portion (138) is defined by an outer tapered sidewall (138a) and the inner tapered portion (139)is defined by an inner tapered sidewall (139a), wherein the outer tapered sidewall (138a) and the inner tapered sidewall (139a) together define a double taper.

9. The prosthetic device of any preceding claim, wherein the modular neck component (12) further comprises an anteverted portion (136) located near the distal end (34) of the modular neck component, and preferably wherein the anteverted portion (136) is formed such that the modular neck component (12) is positioned within the recess (120) of the stem component at an anteversion angle that is defined between a longitudinal axis of the stem component and a neck axis, when the modular neck component is positioned within the recess, that is between the range of about zero and about twenty degrees, preferably about ten degrees.

10. The prosthetic device of any preceding claim, wherein the outer tapered portion (138) comprises a diameter that is greater than a diameter of the inner tapered portion (139).

11. The prosthetic device of any preceding claim, wherein the modular neck component (12) and the stem component (11) are manufactured from a cobalt-chrome alloy.

12. The prosthetic device of claims 2 to 11, wherein the proximal conical flare (50) extends outwardly in an anterior, posterior, and medial directions, and preferably wherein the contour of the proximal conical flare has a symmetrical taper ratio per each side of the proximal conical flare.

13. The prosthetic device of any preceding claim, wherein the stem component (11) has a longitudinal axis that is centered with respect to a distal portion of the stem component, wherein a plane runs through the longitudinal axis and separates an anterior side and a posterior side of said stem component;
wherein the device further comprises an anterior metaphyseal tapering flare (80) located anteriorly on the proximal portion of the stem component (11) such that a surface area of the anterior side of the proximal portion is greater than a surface area of the posterior side of the proximal portion, wherein the anterior metaphyseal tapering flare is configured for providing solid contact with an anterior portion of cortical bone to thereby transfer stress from the device to the bone.

14. The prosthetic device of claim 13, wherein the anterior metaphyseal tapering flare (80) further comprises a surface (82) that has a taper angle relative to a line parallel to the longitudinal axis of the stem component, the taper angle being within a range of about ten degrees to about twenty degrees, preferably about twelve degrees to about sixteen degrees, and further preferably wherein the taper angle is fourteen degrees.

15. The prosthetic device of claim 14, wherein the anterior metaphyseal tapering flare (80) further comprises an enlarged portion (81) that protrudes from the anterior side of the proximal portion (14) configured as an anatomical body to engage the cortical bone to thereby transfer stress from the device to the bone.

16. The prosthetic device of claim 14, wherein the anterior metaphyseal tapering flare (80) further comprises a surface (82) that begins to taper from the proximal end of the stem component distally toward the distal end of the stem component (11) for the length and meets with the proximal portion at a junction, wherein the length is approximately one-half of a length of the entire proximal portion, and preferably wherein the proximal portion further comprises a second surface extending beyond the junction that tapers at an angle relative to the longitudinal axis of the stem component, the taper angle being within a range of about three degrees to about six degrees, preferably wherein the taper angle is four degrees.

17. The prosthetic device of any preceding claim, wherein the modular neck component (12) is manufactured from cobalt-chromium-molybdenum alloy, and the stem component (11) is manufactured from titanium alloy.

18. The prosthetic device of claim 17, wherein the stem component (11) comprises symmetrical, straight sides such that the stem component has a substantially uniform cross section.

19. The prosthetic device of any preceding claim, wherein the proximal portion of the stem component (11) comprises a surficial roughness (116) configured for increased interdigitation between the proximal portion and at least one of the following, bone and bone cement, and preferably wherein the device further comprises a restrictor (115) having an exterior surface and a depression therein such that the restrictor has bowl shape, wherein the restrictor may at least partially surround the stem component such that said restrictor acts to restrict bone cement from flowing beneath the restrictor, and further preferably wherein the restrictor is positioned in engagement with the stem component near a mid-portion of said stem component such that said restrictor separates the proximal portion from a distal portion of the stem component, and is configured and dimensioned to centralize the stem
component within the cavity of the bone to thereby maintain the stem component from slipping into a varus and valgus position, and also further preferably, wherein the restrictor is manufactured from a thermoplastic material.

20. The prosthetic device of any preceding claim, wherein the stem component further comprises a slot (60) formed within a distal portion of the stem component, the slot being defined by an opposing first inner wall and a second inner wall, and preferably wherein the slot is formed as a coronal slot (60), and further preferably wherein the slot is formed as a sagittal slot, or further preferably wherein the slot is formed as a twisted slot, and preferably wherein the slot twists in a partial helix about a longitudinal axis of the stem component, more preferably wherein the stem component simulates the physiological twisting and bending of the bone due to the twisting configuration of the slot, and further preferably wherein the opposing first and second inner walls of the slot twist around the distal portion on an anterior side, a posterior side, and a lateral side of the distal portion.

21. The prosthetic device of any preceding claim, wherein the stem component comprises a flat anterior surface, posterior surface, medial surface and lateral surface, wherein the stem component is substantially shaped as a wedge.

22. The prosthetic device of any preceding claim, wherein the distal portion comprises at least one flute (43).

23. The prosthetic device of any preceding claim, wherein the proximal end of the modular neck component is configured for attachment to a head component of the prosthetic device; and
wherein the stem component (11) is configured for implantation into a canal of the bone and further comprises a distal portion, wherein the recess (120) is defined by a first and second sidewall;
wherein the outer tapered portion (138) of the modular neck component is defined by an outer tapered sidewall (138a) and the inner tapered portion (139) of the modular neck component is defined by an inner tapered sidewall (139a), and together the outer tapered sidewall and the inner tapered sidewall define a double taper, such that the outer tapered sidewall and the inner tapered sidewall of the modular neck component matingly engage the first and second sidewall of the recess in a friction fit to thereby attach the modular neck component to the stem component, and preferably wherein the engagement between the inner tapered sidwall of the modular neck component and the second sidewall of the recess forms a primary self-locking taper fit, further preferably wherein the engagement between the outer tapered sidewall of the modular neck component and the first sidewall of the recess forms a secondary locking fit that serves as an emergency backup should the primary self-locking taper fail, and also preferably wherein the inner tapered portion of the modular neck has a length that is about one to about ten times a length of the outer tapered portion of the modular neck, wherein the inner portion is configured and dimensioned so as to avoid bottoming out in the recess thereby allowing the friction fit to occur, and preferably wherein the length of the inner tapered portion is about three to about four times the length of the outer tapered portion.

24. The prosthetic device of claim 23, wherein the outer tapered portion (138) comprises a diameter and the inner tapered portion (139) comprises a diameter, wherein the diameter of the outer portion is greater than the diameter of the inner tapered portion.

25. The prosthetic device of claim 23, wherein the outer tapered sidewall (138a) and the inner tapered sidewall (139a) of the modular neck both taper at an angle relative to a longitudinal reference axis of the modular neck component (12), the angle being within a range of self-locking taper angles, and preferably wherein the first and second sidewall of the recess both taper at an angle relative to a neck axis, the angle being within a range of taper angles of the self-locking type, such that the inner tapered sidewall and the second sidewall form a primary self-locking taper fit, and the outer tapered sidewall and the first sidewall form a secondary self-locking taper fit that serves as an emergency backup should the primary self-locking taper fail.

## Patentansprüche

1. Prothetische Vorrichtung (10) zur Implantation in einen Knochen, wobei die Vorrichtung aufweist:
eine modulare Halskomponente (12), die ein proximales Ende (32), ein distales Ende (34) und einen äußeren abgeschrägten Teil (138) aufweist, der sich unterhalb des distalen Endes der modularen Halskomponente erstreckt; und
eine Schaftkomponente (11) mit einer Vertiefung (120), die in einem proximalen Teil (14) davon gebildet ist, wobei die Vertiefung einen ersten Teil, der durch eine erste abgeschrägte Seitenwand (140) definiert ist, und einen zweiten Teil hat, der durch eine zweite abgeschrägte Seitenwand (142) definiert ist; wobei der proximale Teil (14) der Schaftkomponente (11) sich nach außen in eine distale zu proximale Richtung in eine äußere Aufweitung aufweitet, um dadurch einen aufgeweiteten Oberflächenteil zu bilden, der einen Aufweitungswinkel bezüglich einer longitudinalen Achse der Schaftkomponente innerhalb eines Bereichs von in etwa fünfzehn bis in etwa fünfundvierzig Grad bildet,
**dadurch gekennzeichnet, dass** die modulare Halskomponente einen inneren abgeschrägten Teil (139) aufweist, der sich distal unterhalb des äußeren abgeschrägten Teils (138) erstreckt, und dadurch, dass der äußere abgeschrägte Teil (138) der modularen Halskomponente (12) eine Vielzahl von ersten Keilen (124) aufweist, die innerhalb eines Umfangs des äußeren abgeschrägten Teils definiert sind, und wobei die erste Seitenwand (140) des ersten Teils der Vertiefung (120) eine Vielzahl von entsprechenden zweiten Keilen (122) zum passenden Erfassen der Vielzahl der ersten Keile (124) der modularen Halskomponente (12) aufweist, um dadurch die modulare Halskomponente in vielen, vorbestimmten Orientierungen innerhalb der Vertiefung (120) des proximalen Teils (14) der Schaftkomponente (11) zu positionieren.

2. Prothetische Vorrichtung nach Anspruch 1, wobei die äußere Aufweitung des proximalen Teils (14) der Schaftkomponente eine proximale konische Aufweitung (50) mit einer Unteroberfläche (54) mit einer Kontur ist, die in einer gerundeten konischen Weise geformt ist, sodass die proximale konische Aufweitung wenigstens einen Teil einer proximalen metaphysären Höhlung in dem Knochen füllt, wobei die proximale konische Aufweitung zum Verursachen einer Druckbelastung auf den Knochen gestaltet ist, sodass die proximale konische Aufweitung (50) hilft unnatürlicher Umfangsspannungen, die auf die Vorrichtung (10) ausgeübt werden, in natürlichere Kompressionsdrücke zu transferieren;

3. Prothetische Vorrichtung nach Anspruch 1 oder 2, wobei der Aufweitungswinkel ausgewählt ist aus in etwa achtzehn Grad, in etwa zwanzig Grad, in etwa zweiundzwanzig Grad, in etwa vierundzwanzig Grad, in etwa sechsundzwanzig Grad, in etwa achtundzwanzig Grad, in etwa dreißig Grad, in etwa zweiunddreißig Grad, in etwa vierunddreißig Grad, in etwa sechsunddreißig Grad, in etwa achtunddreißig Grad, in etwa vierzig Grad, in etwa zweiundvierzig Grad, oder in etwa vierundvierzig Grad.

4. Prothetische Vorrichtung nach einem der vorherigen Ansprüche, wobei die Schaftkomponente (11) eine longitudinale Achse hat, die in Bezug auf den distalen Teil der Schaftkomponente zentriert ist, wobei eine Fläche durch die longitudinale Achse verläuft und eine anteriore Seite (18) und eine posteriore Seite (19) der Schaftkomponente (11) trennt und wobei die proximale konische Aufweitung (50) weiter eine Oberfläche (56) aufweist, die sich abschrägt und eine posteriore Aufweitung (57) bildet, die proximal auf der posterioren Seite (19) der Schaftkomponente (11) liegt.

5. Prothetische Vorrichtung nach Anspruch 4, wobei die Oberfläche nicht konvex ist und die nicht konvexe Oberfläche sich bevorzugt abschrägt, ohne sich auch durch einen ansteigenden und absteigenden Bereich von verschiedenen Winkeln zu erstrecken.

6. Prothetische Vorrichtung nach einem der Ansprüche 2 bis 5, wobei die proximale konische Aufweitung (50) weiter einen posterioren Radius (250) aufweist, der als eine Entfernung zwischen einem Punkt (251), der in Bezug auf die Vertiefung (120) der Schaftkomponente mittig ist, und einem Ende (250a) der proximalen konischen Aufweitung definiert ist, das an der posterioren Kante der proximalen konischen Aufweitung liegt, sodass der posteriore Radius größer ist als ein anteriorer Radius.

7. Prothetische Vorrichtung nach einem der Ansprüche 2 bis 6, wobei die proximale konische Aufweitung (50) in einem obersten Stück des proximalen Teils (14) der Schaftkomponente (11) gebildet ist und in etwa ein Prozent bis in etwa zwanzig Prozent der Gesamtlänge der Schaftkomponente, bevorzugt in etwa vier Prozent bis in etwa zehn Prozent der Gesamtlänge der Schaftkomponente und weiter bevorzugt in etwa sechs Prozent der Gesamtlänge der Schaftkomponente aufweist.

8. Prothetische Vorrichtung nach einem der vorherigen Ansprüche, wobei der äußere abgeschrägte Teil (138) durch eine äußere abgeschrägte Seitenwand (138a) definiert ist und der innere abgeschrägte Teil (139) durch eine innere abgeschrägte Seitenwand (139a) definiert ist, wobei die äußere abgeschrägte Seitenwand (138a) und die innere abgeschrägte Seitenwand (139a) zusammen eine doppelte Abschrägung definieren.

9. Prothetische Vorrichtung nach einem der vorherigen Ansprüche, wobei die modulare Halskomponente (12) weiter einen antevertierten Teil (136) aufweist, der in der Nähe des distalen Endes (34) der modularen Halskomponente liegt, und wobei bevorzugt der antevertierte Teil (136) so gebildet ist, dass die modulare Halskomponente (12) innerhalb der Vertiefung (120) der Schaftkomponente bei einem Anteversionswinkel positioniert ist, der zwischen einer longitudinalen Achse der Schaftkomponente und einer Halsachse definiert ist, wenn die modulare Halskomponente innerhalb der Vertiefung positioniert ist und der in dem Bereich zwischen in etwa null und in etwa zwanzig Grad, bevorzugt bei in etwa zehn Grad liegt.

10. Prothetische Vorrichtung nach einem der vorherigen Ansprüche, wobei der äußere abgeschrägte Teil (138) einen Durchmesser aufweist, der größer ist als ein Durchmesser des inneren abgeschrägten Teils (139).

11. Prothetische Vorrichtung nach einem der vorherigen Ansprüche, wobei die modulare Halskomponente (12) und die Schaftkomponente (11) aus einer Kobalt-Chrom-Legierung gefertigt sind.

12. Prothetische Vorrichtung nach einem der Ansprüche 2 bis 11, wobei die proximale konische Aufweitung (50) sich nach außen in eine anteriore, posteriore und mediale Richtung erstreckt und wobei die Kontur der proximalen konischen Aufweitung bevorzugt ein symmetrisches Abschrägungsverhältnis für jede Seite der proximalen konischen Aufweitung hat.

13. Prothetische Vorrichtung nach einem der vorherigen Ansprüche, wobei die Schaftkomponente (11) eine Longitudinalachse hat, die in Bezug auf einen distalen Teil der Schaftkomponente zentriert ist, wobei eine Fläche durch die longitudinale Achse verläuft und eine anteriore Seite und eine posteriore Seite der Schaftkomponente trennt;
wobei die Vorrichtung weiter eine anteriore metaphysär abgeschrägte Aufweitung (80) aufweist, die anterior auf dem proximalen Teil der Schaftkomponente (11) liegt, sodass ein Oberflächengebiet der anterioren Seite des proximalen Teils größer ist als ein Oberflächengebiet der posterioren Seite des proximalen Teils, wobei die anteriore metaphysär abgeschrägte Aufweitung zum Bereitstellen eines stabilen Kontakts mit einem anterioren Teil eines kortikalen Knochens gestaltet ist, um dadurch Spannungen von der Vorrichtung auf den Knochen zu transferieren.

14. Prothetische Vorrichtung nach Anspruch 13, wobei die anteriore metaphysär abgeschrägte Aufweitung (80) weiter eine Oberfläche (82) aufweist, die einen Abschrägungswinkel bezüglich einer Linie parallel zu der longitudinalen Achse der Schaftkomponente hat, wobei der Abschrägungswinkel innerhalb eines Bereichs von in etwa zehn Grad bis in etwa zwanzig Grad, bevorzugt in etwa zwölf Grad bis in etwa sechzehn Grad liegt und wobei weiter bevorzugt der Abschrägungswinkel vierzehn Grad beträgt.

15. Prothetische Vorrichtung nach Anspruch 14, wobei die anteriore metaphysär abgeschrägte Aufweitung (80) weiter einen vergrößerten Teil (81) aufweist, der aus der anterioren Seite des proximalen Teils (14) herausragt und der als ein anatomischer Körper zum Erfassen des kortikalen Knochens gestaltet ist, um dadurch Spannung von der Vorrichtung auf den Knochen zu transferieren.

16. Prothetische Vorrichtung nach Anspruch 14, wobei die anteriore metaphysär abgeschrägte Aufweitung (80) weiter eine Oberfläche (82) aufweist, die sich beginnend am proximalen Ende der Schaftkomponente distal bis zu dem distalen Ende der Schaftkomponente (11) auf der Länge abschrägt und sich mit dem proximalen Teil an einer Verbindungstelle trifft, wobei die Länge ca. eine Hälfte einer Länge des gesamten proximalen Teils beträgt und wobei der proximale Teil bevorzugt weiter eine zweite Oberfläche aufweist, die sich über die Verbindungstelle hinaus erstreckt und die sich bei einem Winkel bezüglich der longitudinalen Achse der Schaftkomponente abschrägt, wobei der Abschrägungswinkel innerhalb eines Bereichs von in etwa drei Grad bis in etwa sechs Grad liegt, wobei bevorzugt der Abschrägungswinkel vier Grad beträgt.

17. Prothetische Vorrichtung nach einem der vorherigen Ansprüche, wobei die modulare Halskomponente (12) aus einer Kobalt-Chrom-Molybdän-Legierung gefertigt ist und die Schaftkomponente (11) aus einer Titanlegierung gefertigt ist.

18. Prothetische Vorrichtung nach Anspruch 17, wobei die Schaftkomponente (11) symmetrische, gerade Seiten aufweist, sodass die Schaftkomponente einen im Wesentlichen gleichförmigen Querschnitt hat.

19. Prothetische Vorrichtung nach einem der vorherigen Ansprüche, wobei der proximale Teil der Schaftkomponente (11) eine oberflächliche Rauheit (116) aufweist, die zur zunehmenden Verzahnung zwischen dem proximalen Teil und wenigstens einem der Folgenden, Knochen und Knochenzement, gestaltet ist, und wobei die Vorrichtung bevorzugt weiter einen Begrenzer (115) mit einer exterioren Oberfläche und einer Senkung darin aufweist, sodass der Begrenzer eine Schalenform hat, wobei der Begrenzer wenigstens teilweise die Schaftkomponente umgeben könnte, sodass der Begrenzer wirkt, um Fließen von Knochenzement unter dem Begrenzer einzuschränken, und wobei weiter bevorzugt der Begrenzer in der Nähe eines Mittelteils der Schaftkomponente in Verbindung mit der Schaftkomponente positioniert ist, sodass der Begrenzer den proximalen Teil von einem distalen Teil der Schaftkomponente trennt und zum Zentralisieren der Schaftkomponente in der Höhlung des Knochens gestaltet und dimensioniert ist, um dadurch die Schaftkomponente beim Gleiten in eine varisierende und valgisierende Position zu unterstützen, und wobei ebenso weiter bevorzugt der Begrenzer aus einem thermoplastischen Material gefertigt ist.

20. Prothetische Vorrichtung nach einem der vorherigen Ansprüche, wobei die Schaftkomponente weiter einen Spalt (60) aufweist, der innerhalb eines distalen Teils der Schaftkomponente gebildet ist, wobei der Spalt durch eine gegenüberliegende erste innere Wand und eine zweite innere Wand definiert ist und wobei der Spalt bevorzugt als ein Koronalspalt (60) gebildet ist und wobei weiter bevorzugt der Spalt als ein Sagittalspalt gebildet ist oder wobei weiter bevorzugt der Spalt als ein gewundener Spalt gebildet ist und wobei bevorzugt der Spalt sich in einer teilweisen Helix um eine longitudinale Achse der Schaftkomponente windet, wobei insbesondere bevorzugt die Schaftkomponente die physiologische Windung und Biegung des Knochens aufgrund der gewundenen Gestaltung des Spaltes nachbildet und wobei weiter bevorzugt die gegenüberliegenden ersten und zweiten inneren Wände des Spalts sich um den distalen Teil auf einer anterioren Seite, einer posterioren Seite und einer lateralen Seite des distalen Teils winden.

21. Prothetische Vorrichtung nach einem der vorherigen Ansprüche, wobei die Schaftkomponente eine flache anteriore Oberfläche, posteriore Oberfläche, mediale Oberfläche und laterale Oberfläche aufweist, wobei die Schaftkomponente im Wesentlichen als ein Keil geformt ist.

22. Prothetische Vorrichtung nach einem der vorherigen Ansprüche, wobei der distale Teil wenigstens eine Furche (43) aufweist.

23. Prothetische Vorrichtung nach einem der vorherigen Ansprüche, wobei das proximale Ende der modularen Halskomponente zum Anbringen an eine Kopfkomponente der prothetischen Vorrichtung gestaltet ist; und
wobei die Schaftkomponente (11) zur Implantation in einen Kanal des Knochens gestaltet ist und weiter einen distalen Teil aufweist, wobei die Vertiefung (120) durch eine erste und zweite Seitenwand definiert ist;
wobei der äußere abgeschrägte Teil (138) der modulare Halskomponente durch eine äußere abgeschrägte Seitenwand (138a) definiert ist und der innere abgeschrägte Teil (139) der modularen Halskomponente durch eine innere abgeschrägte Seitenwand (139a) definiert ist und die äußere abgeschrägte Seitenwand und die innere abgeschrägte Seitenwand zusammen eine doppelte Abschrägung definieren, sodass die äußere abgeschrägte Seitenwand und die innere abgeschrägte Seitenwand der modularen Halskomponente die erste und zweite Seitenwand der Vertiefung in einer Friktionspassung passend erfassen, um dadurch die modulare Halskomponente an die Schaftkomponente anzubringen, und wobei bevorzugt die Verbindung zwischen der inneren abgeschrägten Seitenwand der modularen Halskomponente und der zweiten Seitenwand der Vertiefung eine primäre selbstsicherende abgeschrägte Passung bildet, wobei weiter bevorzugt die Verbindung zwischen der äußeren abgeschrägten Seitenwand der modularen Halskomponente und der ersten Seitenwand der Vertiefung eine sekundäre Sicherungspassung bildet, die als ein Notfallbackup dient sollte die primäre selbstsichernde Abschrägung versagen, und wobei ebenfalls bevorzugt der innere abgeschrägte Teil des modularen Halses eine Länge hat, die in etwa ein- bis zehnmal einer Länge des äußeren abgeschrägten Teils des modularen Halses beträgt, wobei der innere Teil gestaltet und dimensioniert ist, um ein Durchschlagen in die Vertiefung zu verhindern, wodurch zugelassen wird, dass die Friktionspassung auftritt, und wobei bevorzugt die Länge des inneren abgeschrägten Teils in etwa drei- bis viermal der Länge des äußeren abgeschrägten Teils beträgt.

24. Prothetische Vorrichtung nach Anspruch 23, wobei der äußere abgeschrägte Teil (138) einen Durchmesser aufweist und der innere abgeschrägte Teil (139) einen Durchmesser aufweist, wobei der Durchmesser des äußeren Teils größer ist als der Durchmesser des inneren abgeschrägten Teils.

25. Prothetische Vorrichtung nach Anspruch 23, wobei die äußere abgeschrägte Seitenwand (138a) und die innere abgeschrägte Seitenwand (139a) des modularen Halses sich beide in einem Winkel bezüglich einer longitudinalen Referenzachse der modularen Halskomponente (12) abschrägen, wobei der Winkel innerhalb eines Bereichs von selbstsichernden Abschrägungswinkeln liegt und wobei sich bevorzugt die erste und zweite Seitenwand der Vertiefung beide in einem Winkel bezüglich einer Halsachse abschrägen, wobei der Winkel innerhalb eines Bereichs von Abschrägungswinkeln des selbstsichernden Typs befindet, sodass die innere abgeschrägte Seitenwand und die zweite Seitenwand eine primäre selbstsichernde abgeschrägte Passung bilden und die äußere abgeschrägte Seitenwand und die erste Seitenwand eine sekundäre selbstsichernde abgeschrägte Passung bilden, die als ein Notfallbackup dient, sollte die primäre selbstsichernde Abschrägung versagen.

## Revendications

1. Dispositif prosthétique (10) pour une implantation dans un os, le dispositif comprenant :
un composant de col modulaire (12) comprenant une extrémité proximale (32), une extrémité distale (34) et une partie effilée externe (138), s'étendant en dessous de l'extrémité distale du composant de col modulaire ; et
un composant de tige (11) ayant un évidement (120) formé dans une partie proximale (14) de ce dernier, ledit évidement ayant une première partie définie par une première paroi latérale effilée (140) et une seconde partie définie par une seconde paroi latérale effilée (142) ; dans lequel la partie proximale (14) dudit composant de tige (11) s'évase vers l'extérieur dans une direction distale-proximale selon un évasement vers l'extérieur de sorte à former, de ce fait, une partie de surface évasée qui forme un angle d'évasement par rapport à un axe longitudinal du composant de tige dans une plage allant d'environ quinze à environ quarante-cinq degrés,
**caractérisé en ce que** le composant de col modulaire comprend une partie effilée interne (139) s'étendant distalement en dessous de la partie effilée externe (138) et **en ce que** la partie effilée externe (138) du composant de col modulaire (12) comprend une pluralité de premières cannelures (124), définies dans un périmètre de la partie effilée externe, et dans lequel la première paroi latérale (140) de la première partie de l'évidement (120) comprend une pluralité de secondes cannelures correspondantes (122) pour venir en prise par accouplement avec la pluralité de premières cannelures (124) dudit composant de col modulaire (12) afin de positionner ainsi le composant de col modulaire selon de multiples orientations prédéterminées dans ledit évidement (120) de la partie proximale (14) du composant de tige (11).

2. Dispositif prosthétique selon la revendication 1, dans lequel l'évasement vers l'extérieur de la partie proximale (14) du composant de tige est un évasement conique proximal (50) ayant une surface inférieure (54) ayant un contour qui est formé de manière conique arrondie de telle sorte que l'évasement conique proximal remplisse au moins une partie d'une cavité métaphysaire proximale dans l'os, dans lequel ledit évasement conique proximal est configuré pour provoquer une charge de compression sur l'os de telle sorte que l'évasement conique proximal (50) aide à transférer des contraintes périphériques non naturelles exercées sur le dispositif (10) en charges compressives plus naturelles.

3. Dispositif prosthétique selon la revendication 1 ou 2, dans lequel l'angle d'évasement est sélectionné parmi environ dix-huit degrés, environ vingt degrés, environ vingt-deux degrés, environ vingt-quatre degrés, environ vingt-six degrés, environ vingt-huit degrés, environ trente degrés, environ trente-deux degrés, environ trente-quatre degrés, environ trente-six degrés, environ trente-huit degrés, environ quarante degrés, environ quarante-deux degrés ou environ quarante-quatre degrés.

4. Dispositif prosthétique selon l'une quelconque des revendications précédentes, dans lequel le composant de tige (11) possède un axe longitudinal qui est centré par rapport à la partie distale du composant de tige, dans lequel un plan s'étend à travers l'axe longitudinal et sépare un côté antérieur (18) et un côté postérieur (19) dudit composant de tige (11), et dans lequel l'évasement conique proximal (50) comprend en outre une surface (56) qui s'effile, formant un évasement postérieur (57) qui est situé proximalement sur le côté postérieur (19) du composant de tige (11).

5. Dispositif prosthétique selon la revendication 4, dans lequel la surface est non convexe et, de préférence, la surface non convexe s'effile sans également s'étendre grâce à une gamme croissante et décroissante d'angles variables.

6. Dispositif prosthétique selon les revendications 2 à 5, dans lequel l'évasement conique proximal (50) comprend en outre un rayon postérieur (250) défini comme étant une distance entre un point (251) qui est central par rapport à l'évidement (120) du composant de tige et une extrémité (250a) de l'évasement conique proximal situé sur le bord postérieur de l'évasement conique proximal de telle sorte que le rayon postérieur soit plus important qu'un rayon antérieur.

7. Dispositif prosthétique selon les revendications 2 à 6, dans lequel l'évasement conique proximal (50) est formé dans une partie la plus haute de la partie proximale (14) du composant de tige (11) et comprend entre environ un pour cent et environ vingt pour cent de toute la longueur du composant de tige, de préférence entre environ quatre pour cent et environ dix pour cent de toute la longueur du composant de tige, et de manière davantage préférée environ six pour cent de toute la longueur du composant de tige.

8. Dispositif prosthétique selon l'une quelconque des revendications précédentes, dans lequel la partie effilée externe (138) est définie par une paroi latérale effilée externe (138a) et la partie effilée interne (139) est définie par une paroi latérale effilée interne (139a), dans lequel la paroi latérale effilée externe (138a) et la paroi latérale effilée interne (139a) définissent ensemble un double cône.

9. Dispositif prosthétique selon l'une quelconque des revendications précédentes, dans lequel le composant de col modulaire (12) comprend en outre une partie antévertie (136) située près de l'extrémité distale (34) du composant de col modulaire et, de préférence, dans lequel la partie antévertie (136) est formée de telle sorte que le composant de col modulaire (12) soit positionné dans l'évidement (120) du composant de tige selon un angle d'antéversion qui est défini entre un axe longitudinal du composant de tige et un axe de col, lorsque le composant de col modulaire est positionné dans l'évidement, qui se situe dans la plage allant d'environ zéro à environ vingt degrés, de préférence est d'environ dix degrés.

10. Dispositif prosthétique selon l'une quelconque des revendications précédentes, dans lequel la partie effilée externe (138) comprend un diamètre qui est plus important qu'un diamètre de la partie effilée interne (139).

11. Dispositif prosthétique selon l'une quelconque des revendications précédentes, dans lequel le composant de col modulaire (12) et le composant de tige (11) sont fabriqués à partir d'un alliage de cobalt-chrome.

12. Dispositif prosthétique selon les revendications 2 à 11, dans lequel l'évasement conique proximal (50) s'étend vers l'extérieur dans des directions antérieure, postérieure et médiale et, de préférence, dans lequel le contour de l'évasement conique proximal présente un rapport d'effilement symétrique par chaque côté de l'évasement conique proximal.

13. Dispositif prosthétique selon l'une quelconque des revendications précédentes, dans lequel le composant de tige (11) présente un axe longitudinal qui est centré par rapport à une partie distale du composant de tige, dans lequel un plan s'étend à travers l'axe longitudinal et sépare un côté antérieur et un côté postérieur dudit composant de tige ;
dans lequel le dispositif comprend en outre un évasement conique métaphysaire antérieur (80) situé antérieurement sur la partie proximale du composant de tige (11) de telle sorte qu'une surface du côté antérieur de la partie proximale soit plus importante qu'une surface du côté postérieur de la partie proximale, dans lequel l'évasement conique métaphysaire antérieur est configuré pour réaliser un contact solide avec une partie antérieure de l'os cortical pour transférer, de ce fait, une contrainte du dispositif à l'os.

14. Dispositif prosthétique selon la revendication 13, dans lequel l'évasement conique métaphysaire antérieur (80) comprend en outre une surface (82) qui présente un angle d'effilement par rapport à une ligne parallèle à l'axe longitudinal du composant de tige, l'angle d'effilement se situant dans une plage allant d'environ dix degrés à environ vingt degrés, de préférence d'environ douze degrés à environ seize degrés, et de manière davantage préférée dans lequel l'angle d'effilement est de quatorze degrés.

15. Dispositif prosthétique selon la revendication 14, dans lequel l'évasement conique métaphysaire antérieur (80) comprend en outre une partie allongée (81) qui fait saillie depuis le côté antérieur de la partie proximale (14) configurée sous la forme d'un corps anatomique pour venir en prise avec l'os cortical pour transférer, de ce fait, une contrainte du dispositif à l'os.

16. Dispositif prosthétique selon la revendication 14, dans lequel l'évasement conique métaphysaire antérieur (80) comprend en outre une surface (82) qui commence à s'effiler depuis l'extrémité proximale du composant de tige distalement vers l'extrémité distale du composant de tige (11) sur toute la longueur et entre en contact avec la partie proximale au niveau d'une jonction, dans lequel la longueur fait approximativement la moitié de la longueur de toute la partie proximale, et de préférence dans lequel la partie proximale comprend en outre une seconde surface s'étendant au-delà de la jonction qui s'effile selon un angle par rapport à l'axe longitudinal du composant de tige, l'angle d'effilement se situant dans la plage allant d'environ trois degrés à environ six degrés, de préférence dans lequel l'angle d'effilement est de quatre degrés.

17. Dispositif prosthétique selon l'une quelconque des revendications précédentes, dans lequel le composant de col modulaire (12) est fabriqué à partir d'un alliage cobalt-chrome-molybdène et le composant de tige (11) est fabriqué à partir d'un alliage de titane.

18. Dispositif prosthétique selon la revendication 17, dans lequel le composant de tige (11) comprend des côtés droits symétriques de telle sorte que le composant de tige présente une section transversale sensiblement uniforme.

19. Dispositif prosthétique selon l'une quelconque des revendications précédentes, dans lequel la partie proximale du composant de tige (11) comprend une rugosité superficielle (116) configurée pour réaliser une meilleure interdigitation entre la partie proximale et au moins l'un parmi l'os et le ciment osseux, et de préférence dans lequel le dispositif comprend en outre un restricteur (115) ayant une surface externe et un creux en son sein de telle sorte que le restricteur présente une forme de bol, dans lequel le restricteur peut entourer au moins partiellement le composant de tige de telle sorte que ledit restricteur serve à limiter l'écoulement du ciment osseux sous le restricteur, et de manière davantage préférée dans lequel le restricteur est positionné en prise avec le composant de tige près d'une partie intermédiaire dudit composant de tige de telle sorte que ledit restricteur sépare la partie proximale d'une partie distale du composant de tige, et est configuré et dimensionné pour centrer le composant de tige dans la cavité de l'os pour ainsi maintenir le composant de tige l'empêchant de glisser dans une position en varus et en valgus, et également de manière davantage préférée dans lequel le restricteur est fabriqué à partir d'un matériau thermoplastique.

20. Dispositif prosthétique selon l'une quelconque des revendications précédentes, dans lequel le composant de tige comprend en outre une fente (60) formée dans une partie distale du composant de tige, la fente étant définie par une première paroi interne et une seconde paroi interne opposées, et de préférence dans lequel la fente est configurée sous la forme d'une fente coronale (60), et de manière davantage préférée dans lequel la fente est configurée sous la forme d'une fente sagittale ou de manière davantage préférée dans lequel la fente est configurée sous la forme d'une fente enroulée et de préférence dans lequel la fente s'enroule selon une hélice partielle autour d'un axe longitudinal du composant de tige, de manière davantage préférée dans lequel le composant de tige simule l'enroulement physiologique et la torsion de l'os en raison de la configuration d'enroulement de la fente et de manière davantage préférée dans lequel les première et seconde parois internes opposées de la fente s'enroulent autour de la partie distale sur un côté antérieur, un côté postérieur et un côté latéral de la partie distale.

21. Dispositif prosthétique selon l'une quelconque des revendications précédentes, dans lequel le composant de tige comprend une surface antérieure plate, une surface postérieure, une surface médiale et une surface latérale, dans lequel le composant de tige a sensiblement la forme d'un coin.

22. Dispositif prosthétique selon l'une quelconque des revendications précédentes, dans lequel la partie distale comprend au moins une cannelure (43).

23. Dispositif prosthétique selon l'une quelconque des revendications précédentes, dans lequel l'extrémité proximale du composant de col modulaire est configurée pour permettre une fixation à un composant principal du dispositif prosthétique ; et
dans lequel le composant de tige (11) est configuré pour permettre une implantation dans un canal de l'os et comprend en outre une partie distale, dans lequel l'évidement (120) est défini par une première et une seconde paroi latérale ;
dans lequel la partie effilée externe (138) du composant de col modulaire est définie par une paroi latérale effilée externe (138a) et la partie effilée interne (139) du composant de col modulaire est définie par une paroi latérale effilée interne (139a) et, ensemble, la paroi latérale effilée externe et la paroi latérale effilée interne définissent un double cône de telle sorte que la paroi latérale effilée externe et la paroi latérale effilée interne du composant de col modulaire viennent en prise par accouplement avec la première et la seconde paroi latérale de l'évidement selon un ajustement par friction pour ainsi fixer le composant de col modulaire au composant de tige, et de préférence dans lequel la mise en prise entre la paroi latérale effilée interne du composant de col modulaire et la seconde paroi latérale de l'évidement forme un ajustement conique primaire à verrouillage automatique, de manière davantage préférée dans lequel la mise en prise entre la paroi latérale effilée externe du composant de col modulaire et la première paroi latérale de l'évidement forme un ajustement de verrouillage secondaire qui fait office d'élément de secours d'urgence si l'élément conique primaire à verrouillage automatique est défaillant, et de préférence également dans lequel la partie effilée interne du col modulaire présente une longueur qui fait environ une à environ dix fois la longueur de la partie effilée externe du col modulaire, dans lequel la partie interne est configurée et dimensionnée de sorte à éviter un blocage dans l'évidement, ce qui permet la réalisation d'un ajustement par friction, et de préférence dans lequel la longueur de la partie effilée interne fait environ trois à environ quatre fois la longueur de la partie effilée externe.

24. Dispositif prosthétique selon la revendication 23, dans lequel la partie effilée externe (138) présente un diamètre et la partie effilée interne (139) présente un diamètre, dans lequel le diamètre de la partie externe est plus important que le diamètre de la partie effilée interne.

25. Dispositif prosthétique selon la revendication 23, dans lequel la paroi latérale effilée externe (138a) et la paroi latérale effilée interne (139a) du col modulaire s'effilent l'une et l'autre selon un angle par rapport à un axe longitudinal de référence du composant de col modulaire (12), l'angle se situant dans une gamme d'angles d'effilement à verrouillage automatique et de préférence dans lequel la première et la seconde paroi latérale de l'évidement s'effilent l'une et l'autre selon un angle par rapport à l'axe du col, l'angle se situant dans une gamme d'angles d'effilement du type à verrouillage automatique de telle sorte que la paroi latérale effilée interne et la seconde paroi latérale forment un ajustement conique primaire à verrouillage automatique, et que la paroi latérale effilée externe et la première paroi latérale forment un ajustement conique secondaire à verrouillage automatique qui fait office d'élément de secours d'urgence si l'élément conique primaire à verrouillage automatique est défaillant.
